# EUROPEAN PATENT APPLICATION

(11) **EP 3 199 644 A1**
(43) Date of publication of application: **02.08.2017**
(21) Application number: 16382028.5
(22) Date of filing: 26.01.2016
(51) Int. Cl.: C12Q 1/68

(54) **METHOD FOR DETECTION OF GENE MUTATIONS COMPRISING THE DETECTION OF DNA FROM EXTRACELLULAR VESICLES**

(71) Applicant: Fundacion de Investigacion HM Hospitales, 28015 Madrid (ES)
(72) Inventor: AYUSO SACIDO, Angel, 28018 Madrid (ES); BELDA INIESTA, Cristobal, 28223 pozuelo de Alarcón (ES); CARRION NAVARRO, Josefa, 28035 Madrid (ES); GARCIA ROMERO, Noemí, 39200 Reinosa, Cantabria (ES)
(74) Representative: Pons

(57) **Abstract**

The present invention describes a method for detecting gene mutations and/or minority alleles comprising extraction the DNA from the total extracellular vesicles isolated from a liquid sample of a subject and amplifying the region of the DNA wherein the gene mutations and/or minority alleles are located by means of a method for enrichment. Preferably the invention refers a method of prognosis of glioma patients by the detection of mutations from IDH1 in serum extracellular vesicles.

SEQUENCE LISTING

<110>  Fundación de Investigación HM Hospitales

<120>  Method for detection of gene mutations comprising the detection
       of DNA from extracellular vesicles

<130>  EP3408.1

<160>  43

<170>  PatentIn version 3.5

<210>  1
<211>  18907
<212>  DNA
<213>  Homo sapiens

<400>  1
ggcggggcct gggaggggac aaagccggga agaggaaaag ctcggaccta ccctgtggtc      60

ccgggtttct gcagagtcta cttcagaagc ggaggcactg ggagtccggt ttgggattgc     120

caggctgtgg ttgtgagtct gagcttgtga gcggctgtgg cgccccaact cttcgccagc     180

atatcatccc ggcaggtaac ctcaggctcc aaggggcggc cccggtccct ggctgtggag     240

gggtggctct aattccgcag aaggcaggaa tggggtaaag gaaaaaagcg cagatcttgg     300

gtgtggaggg agtcaaggcc tgcgtgcaaa gaagggaaca aatccaggta ttgagaatgg     360

gaaaagaaaa gggaaggagg ggatgctgga tgcttggata tgcagaaggt ctgcttttgg     420

gcctgggagg gagaaactgg gcggtgagga agttggatgc aacgacaagg ggaaaatcag     480

gtctgcactt gggttggaaa atcgaggggt ggaagttgca agaatgcatg tagaagacta     540

agcctggagt gtaggaggaa gaaaacgtgc agcagctggg ggctggatgc aaagaaggct     600

gtaagccgca gacgggcgtc tgcaggcaac tcaggcggcg aggaatagcc ttgaagccga     660

tccctcgcgt ccgccagggg gcgtggaggg gcggagctcc agcggccttc agaggggtcg     720

ctaggccaca cggggcgaga gggggctggg ggaaacaccc ccgccagttc cgggtgcctg     780

gagtttaaaa ggtcccagca ccagcgaggg gaggggaggg gaggggcggg cgtggagacc     840

tggaaggagg taccgatcca gccttgattg cagccctctt gcacaacggc cataaggacg     900

ggtgcaggcc aagagcaagg acatgcaaac cccaagacct gctaacgggc ggcgaagcgg     960

gggcacgccc tcgcacacgc agagataaat tgtgctccca tgacctttat ttggaaagtg    1020

cctgcgggcc taaaattggc ctttgtccca ccgagtacac tcagcactgt actttaaacc    1080

ggataaactg ggctgtctgg caggtactaa aaacagctaa attctcctgt gagtgttctg    1140

tatgttaaca cttttattcc ttgttttgtt ttaggcgata aactacattc agttgagtct    1200

gcaagactgg gaggaactgg ggtgataaga aatctattca ctgtcaaggt gagaattagc    1260

aaattttccc ccttcttctg tactttcttt gatgtggcag tacagtgttt aactggtctt    1320

gggggcaggc cgaggcactg caagggaatg agtgaggaat atttccaaca acgaattcca 1380
acagcttcct tgtttctttt aatacagtgt cttatctgca agagatcctt tttgcccaga 1440
aattattgta gttcatgaac tcggcaaaaa acaatctggc caaaaacttg aggttgatga 1500
aaataatatt aaatggctct tttttttcat taaactactc tgtattgtta ttccaggttt 1560
attttctgga attgcacaat tatgttagga gataggattc agctacttca tatgagctgg 1620
tgcctgctgg tcttggaaca gcccttcctt ctgaagttgg gattgggttg gagacaaaat 1680
ctgaggggca acaaaggtgg agaagtcagt ccagtggggc ctcaagccca tcccatggaa 1740
atcagatcca gaagaaatac aattatgttc cacatggtag taacaataaa gcttcactgt 1800
tttagttaaa agatggtaaa aatttcatgg ctaagtaatg tgaggaaaac ctgaagcgtc 1860
tgaagtatgc attatgagat aaagaaagat tactgcagag agtacagttg ctttaactga 1920
gaaggaaata gtgaactaaa aagatatcag tcttcacatt ttaggtacaa tatttgacct 1980
ctattaactg tatttgccat caagaataaa ttgccatgtc atgactgttg catcatacta 2040
tatagggaca tggccatgga agtgtattat caggaaccag ataagttcac tgcctccttt 2100
tttgctttgt tttaaaacaa ggataggtgt gacagtggaa tcaaacatga actgaatcat 2160
atgctttcag ctgtactaca aattatactt gacattacaa atgggaagta attgactcat 2220
ccacaccatt ctgttcattt cttttagtct agttcagttc cagaaggttc ccccaaagga 2280
atctcctaaa cactagtaag aagaattcta gaattgcgaa tttgggaagt gaaaatgggc 2340
ctggggattg gaaatacatc caggcttgtc aagaaacaga cgcttactgt ctactaatca 2400
ttacattttt atttaaagac caaattactt tagctcaatt tttcaagaaa gactatttta 2460
ggcttcttat ctaataattc tttaaagtca attctcttga cttttttagt ccttaagaga 2520
ataaaagcaa tttgcagctg tgttgttgga ttaaaaaaga gtttcgatta gagcatcatt 2580
agaaggaata gataactttt agcaggctca gagcttcttc tgcctctagg gacaagctgg 2640
gacagtttca tttcttccaa agcgtccaac cttgcacttt ctgagaatca gattgatttg 2700
tatatgctac ccccacaccc tatccttaaa gtgctgtctt cctaggacaa tgcacagttt 2760
gtcctatccc actcactagc agcttaccct tgcctgtgaa cagccagaag aagatgacag 2820
ggacatgctt gggtcctggg cagtggccct actgtataga gattcttcta attgagaaat 2880
cactttgttg tatttagccc cacatctcca atgtctgtgt tatctctagg aaaacagttc 2940
caccaggaat tcttcgtctc gcttttagac ttaaagtttc cctgaatatg ggtcacagga 3000
accagctgaa tctcctcatg gctagttatt tttattaaat aacaaaaaca gcctcttaaa 3060
ggaggagaaa tgctagcact caccattctg gacatcttga gagcccagta aaatgatgag 3120
ttggactgaa gtgttcaata aacatgagat tgtggaaata atctcatttc ttaagacaaa 3180
ccagtagttg agaataatga ttgcattatg aagggcacgt tatagggtca ggctctgcca 3240

cgctctagct aggtggcctt gggaaagctc tttttttttt aacaaagtaa aatggggaaa 3300

tgatggccat ttttttggat tgttatgagg gctagctcag aaaccgcgtg tgaaacataa 3360

cacaatactt tgttcagaga agatactcaa ttctaatggt aaatagaatt gctgttacta 3420

gctttttatt caactacaca taaattgaat tgcatgaatt gttcttttag tagtcactac 3480

gttgtttata taaacactac gttgtttata tatttcactt tataatgaaa taaatgacaa 3540

ggcatgtatt ttttttttct ttttaggttt attgaagtca aaatgtccaa aaaaatcagt 3600

ggcggttctg tggtagagat gcaaggagat gaaatgacac gaatcatttg ggaattgatt 3660

aaagagaaac tcatttttcc ctacgtggaa ttggatctac ataggtaaat gagttacccc 3720

tccgtgtagc aaactcagaa aggataatct ggctgggcat ggtggtgcac acgtgaagtc 3780

ccagctactt aggaggcaga ggcaggagga tccctcaaga ccaggaattc aagtccagcc 3840

tgggcaacag agtgagaccc catcacttta aaaaaaaaag gaaaaaaaag ggaaggagaa 3900

acaggaaaaa aaaaaagaat ataatctgaa ttattttgag gtgaagttag ctttttttat 3960

atagatatag ctgtgttttg tgaataaagt ttgttttaag cctaaatata ttcagatgaa 4020

tcttctaatt ttaacatact gctagaaaaa ctggatttat catctaaaag tcaagaatta 4080

taagggaaag aatgattttc agattcttct aaccttctgt ttcctgtgaa ggttttattt 4140

gtaataaaac agtggctaag aatagtacaa tgtattgaga aaagggaaat aagagaggat 4200

gtaagaaaca gtaaactaaa tgcagttaac acaattttgt catttcaaac tcaattttct 4260

atcactttac ttagtcaatg gacctgtttc cttttcatgc ctcacctttt gcccttttca 4320

tacaattgta gtgcagaaag aattcatgat gtttcttagt tgaacccctc ctttccagca 4380

aaattccctt aaatcctgtg tccctttgcc ctgtgtgtaa agtgcctggg gacagatgca 4440

aaagtctatg taattctctc atttttttcg ctgtgttttt cataaacttg cttctgttct 4500

ttgtaccttt aggtagagat agaattttta aaagggtaaa gtggctgaag actggcatag 4560

aaaaatattg agcaacacgt ttcagccaaa ctattggcaa ctctaagtgt gtttgcctca 4620

gcccagaatt agtcatcaaa atttgtccta aggacctgtt tgagtggaga catttaattc 4680

acataatgtc attaaacata ctttttaatt gttctaaacc taagctgctg atgtctatat 4740

tgaaatcatg ataagatata aatacttgca gaagtcaacc aaacataaga aattaggcat 4800

aaaaacatct tccaactagg ttaaaaaaaa aaagcatctt aggaactcct gtttcaggac 4860

tttacatgac agtaggtgaa ctggcagggt tcttttcctt ataatggctg caactaaaga 4920

tgctttgagc ctctttagtg tggaatcatg aggagtttta aaggggagtc actatgacta 4980

ggaggaaaaa atttggtggc tgcagactag ataatttttg gaggctcaaa caagttttaa 5040

atttttccaa aattcctatt attatcactg actggttctt tttcaggaga atggagcagg 5100

tagtttgttt tatgtaggct ggacagtggt ctgggcaatt tgttccttcc tttcttatct 5160
tcttagaaat gttaagaaga caagcaaaaa atctaataat ttgctgatag attctcctta 5220
tgagaaatgg ttcctaggaa cttgaaaact actgatcata cttcctgttg gagttgggat 5280
tggatcaaca tttcctatgc aagaagataa caaaggacat gactttgtta gatttatatg 5340
aattatctaa agtcgaagca gttggaagtt atttttattt ggaaatatgt caagattact 5400
atctctttgt ttgcatcatg agttgggtag gagatctaag aaaggaagat aagggaacca 5460
aaaagaaatc tagaggcagg ttttaagagc tgaaaaacct tttaaatcct tggagaacag 5520
aataaacaaa acattatttt gttttcttgc tttttaacaa actattgcat gcttcaaata 5580
aagctttgca gtcattttct atgctccctg gacaactaac attctttcag ctctgaatga 5640
cctgcaatct ttgtgttggt gtatattaag ctattactct ctggtatgta gaaaaccatt 5700
gatggattcc tttacttacc caataaattg taggcttgtt tccagagcaa acacttgctc 5760
aagaattaca gaaggaatta gctgggcatg gtggtgcatg cctgtagtcc ccgctactgg 5820
ggaggctgag gcgggggaat cacttgaacc caggaaggca gaggttgagt gagccaagat 5880
cgtgccactg cactccagcc tgggcaacaa aacgagactc atctaaaaaa aaaaaaaaaa 5940
gggaaaggct cattttatga ctaactggca ttaatgaagt agtcattcgc atttttggca 6000
tcaactgtca catctttacc tcccaccact tcaacgtcaa aatacaaaaa atcctttaac 6060
ccctcccacc cccttttatt tttaaagagc aatatttact aagggaaaaa ggtggtcctt 6120
agacttacac ttattatact aattatatgg agagaaacca ggccattatt gcctctatct 6180
ggtgaaaaac aggaggcaaa gggcagagtt tgatggtttc cattcatttt cctgatagac 6240
agcttcccat tacaagagga ggggcagccc agcagtctag cctctcttag ttctctttgt 6300
agttggcacc catcttctgt gtttagggtg tgccagtgct aaaacttggc agattttggt 6360
gtactcagag ccttcgcttt ctgcataatg agctctatat gccatcactg cagttgtagg 6420
ttataactat ccatttgtct gaaaaacttt gcttctaatt tttctctttc aagctatgat 6480
ttaggcatag agaatcgtga tgccaccaac gaccaagtca ccaaggatgc tgcagaagct 6540
ataaagaagc ataatgttgg cgtcaaatgt gccactatca ctcctgatga gaagagggtt 6600
gaggagttca agttgaaaca aatgtggaaa tcaccaaatg gcaccatacg aaatattctg 6660
ggtggcacgg tcttcagaga agccattatc tgcaaaaata tcccccggct tgtgagtgga 6720
tgggtaaaac ctatcatcat aggtcgtcat gcttatgggg atcaagtaag tcatgttggc 6780
aataatgtga ttttgcatgt tttttttttc atggcccaga aatttccaac ttgtatgtgt 6840
tttattctta tcttttggta tctacaccca ttaagcaagg tatgaaattg agaaatgcat 6900
atatgtataa ctgtatattt acacacattt agctaaaggc aaatacaaat aaacttacaa 6960
ataggcgtcc atctcaacac attttttttc aaacatgctg ttttttttcc tttatccttt 7020

tattcagtta taccatatga tattgccatt tttatgttgg taatttcata tggttcaacc 7080

agatctgtgg ttttcaacac tggctgcaca ataggatccc cttacaagtt tttttggtgg 7140

ttttgttttg ctttgcttga tttgtttctt tgttttagtt tcaatgcttg agtaccaccc 7200

tacacaaatt aaaatctgaa ttccatgggg ttcaggcatt ttaaagctcc ccaggtgaat 7260

ctaatgtgca aacttgagaa ccaccaaaga ttgtattaaa catgatccca tcatgcataa 7320

aagaaaaaac tggctgggta ctatggttca cacctgtaat cctagcactt tggaggccaa 7380

tttggaagca ctgcttgagg ccaggagttt gagactagcc tggacaacat agcagaaccc 7440

tgtctctaca aaaatgaaaa gtatttcaat aattatgtca attgttcatg gaagagccag 7500

ttttgtttat tcatatacaa agtgagtagg cgaagctgag tggggtggca tgtgcctata 7560

gtcccagcta cttggaaggc tgaggcctga ggatccctta agcctaggag ttcaaggtta 7620

cagctatgag ctatgatcac accactgcct acgcaacaga gcaagaccct gtctcgaaaa 7680

agaaaaagaa aaaaaaaact gactcggtag ttgaaagcag ccttactaaa gcattccttc 7740

ctgcctggaa aaaaagtatt gcttctttac ttctgtacca gtacacttgt tgccaaatta 7800

agcaaaaaaa ctgccaagta aacaaaatca aagttcagtg ttgaaactga ggtttcagac 7860

tttcacagaa agtagtttat gtcttaaaag gaccttaaaa acaggaatct ctcatgctga 7920

aatccagagg ttttaatgta gccatcattg aaaacagtgt gttgagggta aacagtttag 7980

tccttgaggg gtcagttaac tttgttccac tctgtggcta aaactatcct ttcatcctgg 8040

ccaaagcctt cagaaatagc tcccaggggc ccaagcaagg gaaggtagat ggcagtggtg 8100

caaactcgct aatcttgtaa aaatcaccct agattctttc cagttcagtc gtgccttttt 8160

tctgcgtata tcctttttgt tttttgtttt tgagacagag tctcgctctg tcgcccaggc 8220

tggagtgcag tggtgtgatc ttggctcact gcaacctctg cctcccgggt tcaagcaatt 8280

ctcctgcctc agcctcttga gtagctggga ttacaggcac atgctaccat gcccggctaa 8340

tttttgtgtt tttagtagag acggggtttc accatgttgg tcaggctggt ctcaaactcc 8400

tgaccttgtg atccgcccgc ctccgcctcc gcctcccaaa gtgctgggat tacaggcatg 8460

ccaccatgcc cggccttcct cttttttttt ttttttttaa ttattttggc atcagtcatt 8520

tgtaaaaatg gccattctat ttattaaagt ccagaaagag aatttcaaac ctcaggttga 8580

ttcatggact gtttaaatat acataaacct atacaaagca tttatggtgc tttggtacct 8640

cagtttctcc cttttcagaa atagtaataa attaccaccc atatttactt tatggatttt 8700

atatgtcaaa aatgttctgt gtaaagtagg tgtttaatct ttattgttac ttactgctct 8760

tttaccagtt gttattagta tttctctaca cttatagatg tggccggcac ctgctggtac 8820

ctaactctgc aagtatctga ttcaattcat tgaagaagat acaatgcagc aaagacttat 8880

ggatgtctag tatatgccag gcatattatt ccactgagta gctgaggccc atgagggagg 8940

tgcctgtcct tgaacagccc tttcctaaaa atcatcatca tcccattgta gctgctccca 9000

gaatgatttg attgctccat atgaacaagt cctataacac tagtaacaat gataccatga 9060

gagaccgagc cccgatggcc taagtaagga cgagacctca agtcctttga ccctggatgc 9120

attatttcta ctatatcata gttccctcct tgtaagactg atgtaagaat caaatgagat 9180

tatgcaagtt aagcatttaa agcatctggc atagagtaag gacaaagcat tagctacctc 9240

agtactacaa ttagtattgt tatcatcacc ctctagggag acttccctga cttccttcca 9300

tggtcacctc ttttcctctg tcatcctata gttttttttt ttttgggggg ggggtctaat 9360

accctctttt ggcatttgaa agctcttata ttgcttttca cttatatatg tctttatagg 9420

gttgtaaact tcctaaagga aagtggctta tgttattctc tgtcctcttc aaatcagtgc 9480

tcagaaaact ttcctatacc tcgtataggc acctaataaa tatttactga tttaggggaa 9540

tgtctggacc tcttcatccc catctcctaa aaaaaaaaaa aaaaaaagaa gtttgacata 9600

ctgccctatt tgtctaggtg tcttctaggt ctatattata tgaatattct ccttacaatt 9660

cctgctaggg tatttttttt ttctttatac ctttttcccc tctacacagt acagagcaac 9720

tgattttgtt gttcctgggc ctggaaaagt agagataacc tacacaccaa gtgacggaac 9780

ccaaaaggtg acatacctgg tacataactt tgaaggtatg tatgactgta gcataagctt 9840

cttttttttt taaacaaatg atttagataa tatttttatt gctttttttg ttagttatgt 9900

gactgcctaa aaatataatc tgaatctaca gtgtttttgc aaaacccgaa acttgacata 9960

aagaagtaat ttgcttccat accttcctgt ggcatgaata aaggcacaaa agggttaccc 10020

acttaattgg aaatgccttt aggcaaagac aaggagatga ttatgcattt atcttaaagc 10080

acagagagaa ggttggcaga aatctgctgt gcatgaatta tatacattaa atattaatat 10140

tctcaaatca cccagcatat aatcctgccc tttgctctgg tagggctctt gtatctggaa 10200

gaggtttcct tatatatttg ctgaagtttt cttcttgccc ttctgggtat ttatcctgtg 10260

aaaaagtaga acatttgctt tttgcctttt ctttcccagc taccctgcta cctttgatgg 10320

caaatcccac ccaaaagttg ttgaaataat tagtgggccc ttttccttca ctattgaagt 10380

tgttgaaagc actcacacaa acaaaaatac ccaacactca cctacctttt gtttttcttg 10440

cctcttccaa ataatatctg ttggaatctg gttctgcagg cttcagcacc tttcaccctg 10500

aacccaagtt aaccctgagc attggcatgg attcaagttc tatctttaag taataacaaa 10560

gttcaataat agttgtaaaa cacagtgatt acatgtagca taattatttc tacttatttc 10620

taccaactac agtaggagtg ctcagtatcc tccaggagat tataagaatt ttcattcagt 10680

atttgaaatt taagtttttc tcaataactg aggattgagt acacccctaa agtttaaaat 10740

agatgttggg aattgtaaag gtagggtgtt tgtccattct tgcgttgcta taaagaaata 10800

cctgaggctg cgtaagttat aaagaaaaga ggtttaattg gctcatggtt ctgcaggctg 10860

tacaagcctg gctccaacac ctgttcctgg tgagggcctc aggaagcttc caatcttggc 10920

agaaggcaaa cagacaatag gtgtatcaca tggcaagagc gggaacaaga gaaagagaag 10980

ggggaggtcc cagactctta aacaaccaga tttcaagtga attaactgag cgagaactca 11040

cgtaatcacc aaaggggatg gtcctaagcc attcataagg gatccaaccc catgttccaa 11100

tctcctccca ctgggcccca cctccaacac tgggaatcac atttcaacat gagatttgga 11160

agggacaaat atccaaacca tatcagtatg taaaagaagt ttcaaaatta tttgcctttt 11220

cttaaatgta tttctggttc atatcagttt ttggcatttc tgcatcttca ggattttaaa 11280

gactgtatgt tttttcattt ctgctgtcct tccaaaacaa gttagaatgt ccttatctag 11340

aactggcttt tctctcatta tgtgcctcag aaatttgttg gaaacctgtc tgggacttct 11400

gaaatctagt ctcagatgtg agagctgaga agtaggtcat ttggttgtgg tgggtgattt 11460

tagccttatt acaactacat tacatttatt cttctccctt ttttcctctc actggcttct 11520

cctctacaga aggtggtggt gttgccatgg ggatgtataa tcaagataag tcaattgaag 11580

attttgcaca cagttccttc caaatggctc tgtctaaggg ttggcctttg tatctgagca 11640

ccaaaaacac tattctgaag aaatatgatg ggcgttttaa agacatcttt caggagatat 11700

atgacaagta actatagttc ttttttaact ttttctttat tctcaagtaa gctgacattt 11760

gcatatagag tatgtatctc cctatcccta tgattctggg taaattgagt gaaaatcatc 11820

actgtctctt aaagataagg gcaaaactga ggtttagcag gagtgaaaca aaaccactct 11880

gacttgcttt tataagataa acatgttctt aaataaggtc cccacatgaa ttgttttcag 11940

agccaggttc tttaatgttt aaataaacgt acgctgttag tgataagttc taatttttca 12000

tgtgtgagtt tctaagtcag acccatttcc attctttctg aagcccattg ttagaattta 12060

aaattctgtt ggccaggcac ggtggctcat gcctgtaatc ccagcacttt gggaggccga 12120

ggcgggcgga tcacgaggtc aggagatcga gaccatcctg gctaacacgg tgaaaccctg 12180

actctactaa aaataccaaa aattagccgg gcgtggtggc gggcgcctct agtcccagtt 12240

actggggagg ctgaggcagg agaatggcgt gaacccggga ggcggaggtt gcagtgagcc 12300

gaaatcacgc cactgcactg cagcctgggc gacagagcga gactccgtct caaaaaaaaa 12360

aaaagaaaag aaaagaaaaa aaagaattta aaattctgtt ttagtggagt catttgaact 12420

taagtctaag tttataacaa cactggcttc cacagcacag gaggtgagca tgtgttaata 12480

tttaagattg gcataactcc ctttaggtgc aagtgttcag gccaaaatgt tcctgaggca 12540

ttttgattcc tcctcctgct gcccatctat accaagccca gaaactgtct ggaatatatt 12600

ttagtttcct gaatgacacc aagaagtaga acagtctttt caaaaatgta ttttaaaaat 12660

aagctgaatc tcaagaatct gatctatagt ataatgaaaa ctgaaaagtg aagtagtcat 12720

tgggatactc tactgtctca cttaattctc acggcttccc tgcaaggtgg gtaaaattgt 12780

tcctacagat agtcaaattg agttttacag ttagaaaatg attgggctag gatttgagcc 12840

aatgtctgtc agattcctga gtttctgcta cttctactaa aatatgctgc ttcttgtgtg 12900

tccggtcttc tgtttgggac aagcagatga tatccctaac aaaatcaatt ttctttatta 12960

ttattctctt ttaccttttg tttcccaggc agtacaagtc ccagtttgaa gctcaaaaga 13020

tctggtatga gcataggctc atcgacgaca tggtggccca agctatgaaa tcagagggag 13080

gcttcatctg ggcctgtaaa aactatgatg gtgacgtgca gtcggactct gtggcccaag 13140

gtacagaggt ggagcctggc ctgtaggaac agggtcattc cagggtagtt ctctaatttg 13200

ggaaggggag ttttgtagtc ctttgttgta aaatcttgga atgttaaacc tccaggtcca 13260

ccgtcttacc tgacagctaa ggaaattaag ctcagactag agaaatgatt tactcaagac 13320

cacatgggaa gtaaattagt ggattgacac tcctggctcc agtgtcactc cttgaagttt 13380

taaagcctac ctgatctgaa aatcactccc tttccctgcc ctccttgtcc tttggtgaaa 13440

atgtaaaaaa ccagggcact caggcaagga gggataaagt gaggagcttg atcatgtcat 13500

aaccaagctt tggtgcctgc aaagctcatt tattgggcag caggacctac tcatgtccct 13560

ctgccagctg ctgttctggt agaatataaa acaaatgtgt ttggctgggc acagtggctc 13620

acgcctgtaa tcctagcact ttgagaggcc aaggcagtgg atcacttgag gccaggagtt 13680

caagaccagc ctggccaaca tagcaaaacc ccatctctac ttcaaaaaaa aaaaaaaaaa 13740

aaaattagcc aggtgtggtc gcatgcctat agtccctggc actcaggagg ctaaagcatg 13800

agaatttctt gagcctggga agcggaggtt gcagtgagct gagatcacgc cactatactc 13860

cagcctgggc aacagagcaa gactcttgtc tcaacaacaa caaagaaaaa gtgtttttgt 13920

ttactgctta gcttacacac aaatcaattg agcaaagaac cctaggggct gtttacagaa 13980

attccaggag aagtttcatg catggggtaa gggccaaatc aacagaaaca cagcgcctgg 14040

tttcctcagc accaagttgc ccttcagttt cagttgccct tggtagctgt atgaacttga 14100

atctccccag atgaatgttt gttaacttta attatgtttg tttttcccaa gaagtactac 14160

tttttctgtc tcttatcagt gttaaacatc tcctctcagt ttgaaaatca gtagatcaac 14220

ctggttaaag ataacttttt gccagataaa tgagttacac aactctcctt accatccttc 14280

tgtttctcta catcctcaca atcatttgta ctgaatgcaa cagaggaagg agtgttattg 14340

ctttgtgatg actccgtggt tgattattaa cttcacaggt gatgttggga ggtagcaaaa 14400

atggagaaaa gaagaaaatc aattatatag atataagagt tgtaatgtgg ttctttcaat 14460

aagaaaccac agaagagaac tgttgtactg agggtatgaa agagagcgag gaagggcaac 14520

tgggatgttc ttagatccaa ccgatcctgt gtttacctgc cttctcttcc cagtcccagc 14580

aagtgtatga gcagcttgag ggttcttaag agacagctct cgtccatctt tatttcccca 14640

gaacagcaga gcattgcaca taagaataac aataatagat tgagctctgt gtccagtact 14700

attctaagga atataaatat atattcatat atatttatga atatatgtat atttatctaa 14760

tcccacagca acctagtgaa gtaggtgctg ttattactcc cattttacag ataagaaaac 14820

tgagccgcag tagctatgaa gtcatagaac tagagtggaa tgtaggcagt tggaccctga 14880

accctccatc aaggagtagg tatccctgaa ataagtgagg ggaaaatgca gcctttccag 14940

acccattggc tctgatatct ggagcgggga tcctgccttc tgaaaggcag aattattggt 15000

gattccatgt gctcttgttt actttgctta ccaagaccta tcaagattga gtcatttatg 15060

ctctcatctc tgagagactc agtgctcttc atgcagttgg acccaacgct tcatgctcca 15120

ctacttacag ggtatggctc tctcggcatg atgaccagcg tgctggtttg tccagatggc 15180

aagacagtag aagcagaggc tgcccacggg actgtaaccc gtcactaccg catgtaccag 15240

aaaggacagg agacgtccac caatcccatt ggtaagatgg tgtttacatt gatttctcac 15300

cagagagtgc tctccaaagc agcccagatg tgttcccttg gctgctcagt gttttgtgtg 15360

caaagtcatc accaagtagc tgatttgttc tactagtaag atctatttga atatgcttct 15420

taaaatatac aatatttgct tttctgtttt gtgggagaac ataggttaca gagaggaatg 15480

ttatcaacaa gcattttcct ttacctctgg tgcagtgaaa tgtcacataa ggctacaaat 15540

ctaaaagtca catggataga tccttttaac aagtttcaaa gatagcactt caaaaagaga 15600

ttacagcctg gaaaaaggag accaatatag acaagagata gaagagattg tgaccttttt 15660

caaattagaa aatgccttaa gtttcaaaca ctagtaccat gccatgaaaa tgtgtttttt 15720

taaagtttct gtttggtaaa atatttctaa ctctcctaaa acttctagcc catgtggtta 15780

gtatctaaag aagtctgtca cctaaacaaa aaactattct gttttgggga actatgagac 15840

atttggaaac ctctataact aggatgattg gagtgttgtg ttttttttcc cctcttttag 15900

cttccatttt tgcctggacc agagggttag cccacagagc aaagcttgat aacaataaag 15960

agcttgcctt ctttgcaaat gctttggaag aagtctctat tgagacaatt gaggctggct 16020

tcatgaccaa ggacttggct gcttgcatta aaggtttacc caagtaagta taagatgccc 16080

tagtttctat ggtcaaatta acacttatcc tcactgggct cagagcatcg gtgctttctg 16140

gaattatata aattgttatt catcatctag ttcaggagtc agcagatatc tgtaaattgt 16200

cagatagtga acattttagg ctttgtggaa catatagtct ttttgcaact acacaactat 16260

tcaactttgc ctctgccatt gtagcaggaa agcagtcata gacaatacag aatcaaatgg 16320

tgtggctggt accaatcaaa cttttatgtt caaaaccagc ctcgatttgg ttcatgtgct 16380

gggctgtaat ttgctgacat cccatctagt tgataatcat tgaacgaaaa ggttctattt 16440

aattttaaga agcagattat agacaaatag aaacaaagat acttcagagg ctattttatt 16500

ctactccttt attttcttta acttactgat gagaaaagtg agcagcacaa gtttaaggtg 16560

tgcaattgat tacagtccct taagtatctg taaccaatat ttgggcaaat atttgacagc 16620

ccatatgttt actgagaatt tttctgagaa catagtagcc agtatcctgg ggggattgtc 16680

tccatatctc aaccagtgca tctgcagagg tcccaggtag ggtcagtata ccccataatg 16740

gggatatact tggtcactgc atgtttctta atttaaattt ccaacaagat tgcagtgttc 16800

gctgggtgct gtggctcatg cctgtaatcc tagcactttg ggaggccgag gtgggtggat 16860

cacgaggtca ggagatcaag accatcctgg ctaacacagt gaaaccccat ctccactaaa 16920

aatacaaaaa attaaccagg cgtggtggcg ggcacctgta gtcccagcta ctcgggaggc 16980

tgaggcagga gaatggcgtg aacctgggag gcggagcttg cagtgagccg agatggtgcc 17040

actgcactcc agcctgcgtg acagagcgag actccatctc aaaaaaaaaa aaaagattgc 17100

agtgttcaaa atggtcttat cagtggaata cctttgtagc ctatttaaca ggcacctgtc 17160

agcaggatta tgttatcctg tcttttgttt ttcttttttt ttctttttat tgagacagag 17220

tctcgctttg cccaggctgg agtgcagtgg cgtgatcttc gctcactgca acctccgcct 17280

cctgggttca agtgattctt ctgcctcagc ctcccaagta gctgggactt gtgccacaac 17340

acccggctaa tttttttttt tttttttttg tatttttagt agagacgggg tttcactttg 17400

ttagccagga tggtctccat ctcctgacct catgatccac ccgccttggc cccccaaagt 17460

gctgggatta taggcgtgag ccaccaggcc cggcctgtct tttgtttttc ttaagacact 17520

gatagaaata acaacattat aacttttttt aaggacatgt aacggtttgt gggaaggtgg 17580

gtaaaggttt ggctatgccc tgaataagtt ttgttatgtt taagaatgag gaagaagtgt 17640

gatgattttg gtctcagcag cattagtctg aaccgaaact caatgataat ttcaccccca 17700

aagctacata gcaagaaacc taactgagta tgtatggctg tctctactgc cggttgccta 17760

gagagcctga gtactgagcc tgggtcagcc ccaggaaccc tgaacatagt cattgtctat 17820

agattttgta gcttactaac atgaatgcgt tttcttccag aaacaaatag aatactattt 17880

taagtagcat ttctaggact ttaccactac ctgctaccat atcagagacc aactaaattt 17940

tctttttttt ttccttcccg ttagtgtgca acgttctgac tacttgaata catttgagtt 18000

catggataaa cttggagaaa acttgaagat caaactagct caggccaaac tttaagttca 18060

tacctgagct aagaaggata attgtctttt ggtaactagg tctacaggtt tacatttttc 18120

tgtgttacac tcaaggataa aggcaaaatc aattttgtaa tttgtttaga agccagagtt 18180

tatcttttct ataagtttac agcctttttc ttatatatac agttattgcc acctttgtga 18240

acatggcaag ggactttttt acaattttta ttttattttc tagtaccagc ctaggaattc 18300

ggttagtact catttgtatt cactgtcact ttttctcatg ttctaattat aaatgaccaa 18360

aatcaagatt gctcaaaagg gtaaatgata gccacagtat tgctccctaa aatatgcata 18420

aagtagaaat tcactgcctt cccctcctgt ccatgacctt gggcacaggg aagttctggt 18480

gtcatagata tcccgttttg tgaggtagag ctgtgcatta aacttgcaca tgactggaac 18540

gaagtaggag tgcaactcaa atgtgttgaa gatactgcag tcatttttgt aaagaccttg 18600

ctgaatgttt ccaatagact aaatactgtt taggccgcag gagagtttgg aatccggaat 18660

aaatactacc tggaggtttg tcctctccat ttttctcttt ctcctcctgg cctggcctga 18720

atattatact actctaaata gcatatttca tccaagtgca ataatgtaag ctgaatcttt 18780

tttggacttc tgctggcctg ttttatttct tttatataaa tgtgatttct cagaaattga 18840

tattaaacac tatcttatct tctcctgaac tgttgatttt aattaaaatt aagtgctaat 18900

taccatt 18907

<210> 2
<211> 2382
<212> DNA
<213> Homo sapiens

<400> 2
ggcggggcct gggaggggac aaagccggga agaggaaaag ctcggaccta ccctgtggtc 60

ccgggtttct gcagagtcta cttcagaagc ggaggcactg ggagtccggt ttgggattgc 120

caggctgtgg ttgtgagtct gagcttgtga gcggctgtgg cgccccaact cttcgccagc 180

atatcatccc ggcaggcgat aaactacatt cagttgagtc tgcaagactg ggaggaactg 240

gggtgataag aaatctattc actgtcaagg tttattgaag tcaaaatgtc caaaaaaatc 300

agtggcggtt ctgtggtaga gatgcaagga gatgaaatga cacgaatcat ttgggaattg 360

attaaagaga aactcatttt tccctacgtg gaattggatc tacatagcta tgatttaggc 420

atagagaatc gtgatgccac caacgaccaa gtcaccaagg atgctgcaga agctataaag 480

aagcataatg ttggcgtcaa atgtgccact atcactcctg atgagaagag ggttgaggag 540

ttcaagttga aacaaatgtg gaaatcacca aatggcacca tacgaaatat tctgggtggc 600

acggtcttca gagaagccat tatctgcaaa aatatccccc ggcttgtgag tggatgggta 660

aaacctatca tcataggtcg tcatgcttat ggggatcaat acagagcaac tgattttgtt 720

gttcctgggc ctggaaaagt agagataacc tacacaccaa gtgacggaac ccaaaaggtg 780

acatacctgg tacataactt tgaagaaggt ggtggtgttg ccatggggat gtataatcaa 840

gataagtcaa ttgaagattt tgcacacagt tccttccaaa tggctctgtc taagggttgg 900

cctttgtatc tgagcaccaa aaacactatt ctgaagaaat atgatgggcg ttttaaagac 960

atctttcagg agatatatga caagcagtac aagtcccagt ttgaagctca aaagatctgg 1020

tatgagcata ggctcatcga cgacatggtg gcccaagcta tgaaatcaga gggaggcttc 1080

atctgggcct gtaaaaacta tgatggtgac gtgcagtcgg actctgtggc ccaagggtat 1140

ggctctctcg gcatgatgac cagcgtgctg gtttgtccag atggcaagac agtagaagca 1200

gaggctgccc acgggactgt aacccgtcac taccgcatgt accagaaagg acaggagacg 1260

tccaccaatc ccattgcttc catttttgcc tggaccagag ggttagccca cagagcaaag 1320

cttgataaca ataaagagct tgccttcttt gcaaatgctt tggaagaagt ctctattgag 1380

acaattgagg ctggcttcat gaccaaggac ttggctgctt gcattaaagg tttacccaat 1440

gtgcaacgtt ctgactactt gaatacattt gagttcatgg ataaacttgg agaaaacttg 1500

aagatcaaac tagctcaggc caaactttaa gttcatacct gagctaagaa ggataattgt 1560

cttttggtaa ctaggtctac aggtttacat ttttctgtgt tacactcaag gataaaggca 1620

aaatcaattt tgtaatttgt ttagaagcca gagtttatct tttctataag tttacagcct 1680

ttttcttata tatacagtta ttgccacctt tgtgaacatg gcaagggact tttttacaat 1740

ttttatttta ttttctagta ccagcctagg aattcggtta gtactcattt gtattcactg 1800

tcactttttc tcatgttcta attataaatg accaaaatca agattgctca aaagggtaaa 1860

tgatagccac agtattgctc cctaaaatat gcataaagta gaaattcact gccttcccct 1920

cctgtccatg accttgggca cagggaagtt ctggtgtcat agatatcccg ttttgtgagg 1980

tagagctgtg cattaaactt gcacatgact ggaacgaagt aggagtgcaa ctcaaatgtg 2040

ttgaagatac tgcagtcatt tttgtaaaga ccttgctgaa tgtttccaat agactaaata 2100

ctgtttaggc cgcaggagag tttggaatcc ggaataaata ctacctggag gtttgtcctc 2160

tccatttttc tctttctcct cctggcctgg cctgaatatt atactactct aaatagcata 2220

tttcatccaa gtgcaataat gtaagctgaa tcttttttgg acttctgctg gcctgtttta 2280

tttcttttat ataaatgtga tttctcagaa attgatatta aacactatct tatcttctcc 2340

tgaactgttg attttaatta aaattaagtg ctaattacca tt 2382

<210> 3
<211> 414
<212> PRT
<213> Homo sapiens

<400> 3

<210>  4
<211>  20
<212>  DNA
<213>  Artificial Sequence

<220>
<223>  IDH1 primer forward

<400>  4
cggtcttcag agaagccatt                                                   20

<210>  5
<211>  22
<212>  DNA
<213>  Artificial Sequence

<220>
<223>  IDH1 primer reverse

<400>  5
gcaaaatcac attattgcca ac                                                22

<210>  6
<211>  21
<212>  DNA
<213>  Artificial Sequence

<220>
<223>  IDH1 primer forward

<400>  6
gttgaaacaa atgtggaaat c                                                21


<210>  7
<211>  22
<212>  DNA
<213>  Artificial Sequence

<220>
<223>  IDH1 primer reverse

<400>  7
taatgggtgt agataccaaa ag                                               22


<210>  8
<211>  26
<212>  DNA
<213>  Artificial Sequence

<220>
<223>  Primer flanking the IDH1 R132H mutation spot

<400>  8
tgcatttctc aatttcatac cttgct                                           26


<210>  9
<211>  20
<212>  DNA
<213>  Artificial Sequence

<220>
<223>  Primer flanking the IDH1 R132H mutation spot

<400>  9
attctgggtg gcacggtctt                                                  20


<210>  10
<211>  20
<212>  DNA
<213>  Artificial Sequence

<220>
<223>  Primer TP53e7_F

<400>  10
ctttgaggtg cgtgtttgtg                                                  20


<210>  11
<211>  20
<212>  DNA
<213>  Artificial Sequence

<220>
<223>  Primer TP53e7_R

<400>  11
caaccaggag ccattgtctt                                                  20

<210>  12
<211>  21
<212>  DNA
<213>  Artificial Sequence

<220>
<223>  Primer PTENe2_F

<400>  12
gcagaaagac ttgaaggcgt a                                                21


<210>  13
<211>  21
<212>  DNA
<213>  Artificial Sequence

<220>
<223>  Primer PTENi2_R

<400>  13
aaatgaactg taccccctga a                                                21


<210>  14
<211>  21
<212>  DNA
<213>  Artificial Sequence

<220>
<223>  Primer MDM2i1_F

<400>  14
ctcagaatca tcggactcag g                                                21


<210>  15
<211>  20
<212>  DNA
<213>  Artificial Sequence

<220>
<223>  Primer MDM2i3_R

<400>  15
gctgtgtgaa tgcgtcaaat                                                  20


<210>  16
<211>  21
<212>  DNA
<213>  Artificial Sequence

<220>
<223>  Primer MDM4i2_F

<400>  16
atcaggtacg accaaaactg c                                                21


<210>  17
<211>  20

<212>  DNA
<213>  Artificial Sequence

<220>
<223>  Primer MDM4i3_R

<400>  17
ctgggcaaaa gagggagact                                                   20


<210>  18
<211>  18
<212>  DNA
<213>  Artificial Sequence

<220>
<223>  Primer AKT1i12_F

<400>  18
gagggtgtgc tgggagtg                                                     18


<210>  19
<211>  20
<212>  DNA
<213>  Artificial Sequence

<220>
<223>  Primer AKT1e13_R

<400>  19
cttggtcagg tggtgtgatg                                                   20


<210>  20
<211>  21
<212>  DNA
<213>  Artificial Sequence

<220>
<223>  Primer AKT3i13_F

<400>  20
gccgtggaaa cactgaagta a                                                 21


<210>  21
<211>  20
<212>  DNA
<213>  Artificial Sequence

<220>
<223>  Primer AKT3e14_R

<400>  21
gtaggaagcc gactgtggac                                                   20


<210>  22
<211>  20
<212>  DNA
<213>  Artificial Sequence

<220>
<223>  Primer CDK4i2_F

<400>  22
gggttggtag gcattgagag                                                   20


<210>  23
<211>  20
<212>  DNA
<213>  Artificial Sequence

<220>
<223>  Primer CDK4e3_R

<400>  23
tccaccactt gtcaccagaa                                                   20


<210>  24
<211>  20
<212>  DNA
<213>  Artificial Sequence

<220>
<223>  Primer CDKN2Ae2_F

<400>  24
gcaccagagg cagtaaccat                                                   20


<210>  25
<211>  20
<212>  DNA
<213>  Artificial Sequence

<220>
<223>  Primer CDKN2Ai2_R

<400>  25
agggcgatag ggagactcag                                                   20


<210>  26
<211>  20
<212>  DNA
<213>  Artificial Sequence

<220>
<223>  Primer erbb2e25_F

<400>  26
cccttggaca gcaccttcta                                                   20


<210>  27
<211>  20
<212>  DNA
<213>  Artificial Sequence

<220>
<223>  Primer erbb2i25_R

<400> 27
ggtaagcaga cagccacaca 20

<210> 28
<211> 20
<212> DNA
<213> Artificial Sequence

<220>
<223> Primer EGFRi12_F

<400> 28
tgctgtgacc cactctgtct 20

<210> 29
<211> 20
<212> DNA
<213> Artificial Sequence

<220>
<223> Primer EGFRi13_R

<400> 29
caacgcaagg ggattaaaga 20

<210> 30
<211> 20
<212> DNA
<213> Artificial Sequence

<220>
<223> Primer ASCL1i1_F

<400> 30
cctccatctc cctctaccac 20

<210> 31
<211> 20
<212> DNA
<213> Artificial Sequence

<220>
<223> Primer ASCL1e2_R

<400> 31
gactcaggtc ccagttgctc 20

<210> 32
<211> 22
<212> DNA
<213> Artificial Sequence

<220>
<223> Primer MGMTi3_F

<400> 32
tgtgtagatg cgtttcctgt tt 22

<210>  33
<211>  19
<212>  DNA
<213>  Artificial Sequence

<220>
<223>  Primer MGMTe4_R

<400>  33
attgctcctc ccactgctc                                                    19


<210>  34
<211>  20
<212>  DNA
<213>  Artificial Sequence

<220>
<223>  Primer RB1e25_F

<400>  34
ggaagcaacc ctcctaaacc                                                   20


<210>  35
<211>  20
<212>  DNA
<213>  Artificial Sequence

<220>
<223>  Primer RB1i25_R

<400>  35
ccatctcggc tactggaaaa                                                   20


<210>  36
<211>  22
<212>  DNA
<213>  Artificial Sequence

<220>
<223>  Primer PIK3CAe19_F

<400>  36
ggctcaaaga caagaacaaa gg                                                22


<210>  37
<211>  22
<212>  DNA
<213>  Artificial Sequence

<220>
<223>  Primer PIK3CAi19_R

<400>  37
ggaatacaca aacaccgaca ga                                                22


<210>  38
<211>  20

<212>  DNA
<213>  Artificial Sequence

<220>
<223>  Primer Idh1i3_F

<400>  38
aggggaatgt ctggacctct                                                   20


<210>  39
<211>  20
<212>  DNA
<213>  Artificial Sequence

<220>
<223>  Primer Idh1e4_R

<400>  39
tccgtcactt ggtgtgtagg                                                   20


<210>  40
<211>  19
<212>  DNA
<213>  Artificial Sequence

<220>
<223>  Primer IDH2e10_F

<400>  40
gagaaggtgt gcgtggaga                                                    19


<210>  41
<211>  18
<212>  DNA
<213>  Artificial Sequence

<220>
<223>  Primer IDH2i10_R

<400>  41
tcagggatgg ggaggaac                                                     18


<210>  42
<211>  21
<212>  DNA
<213>  Artificial Sequence

<220>
<223>  Primer hmG3PDH_F

<400>  42
acacacagtc catgccatca c                                                 21


<210>  43
<211>  20
<212>  DNA
<213>  Artificial Sequence

<220>
<223>  Primer hmG3PDH_R

<400>  43
tccaccaccc tgttgctgta                                                    20

## Description

The present invention relates to a method for detecting gene mutations and minority alleles that comprises the detection of DNA from extracellular vesicles. Therefore, the present invention relates to the Medicine Field.

### BACKGROUND ART

Malignant gliomas are the most aggressive tumors in the adult brain, being the glioblastoma multiforme (GBM) the most frequent, with an overall survival that rarely exceeds 15 months. GBM has an incidence of two to three per 100,000 adults per year.

The most common type of glioma can be grouped into astrocytoma, oligodendroglioma and oligoastrocytoma, which can be classified based on their histological features (nuclear atypia, number of mitosis, microvascular proliferation and the presence of necrotic regions) into low grade (grades I and II) and high grade (grades III and IV), following the World Health Organization (WHO). Then, astrocytomas include pilocytic (grade I), diffuse (grade II), anaplastic (grade III) and glioblastoma (GBM) (grade IV). The oligodendrogliomas and oligoastrocytomas also include low grade (grade II) and anaplastic (grade III)

In the last years, a number of publications have been published correlating clinical and pathological parameters with molecular alterations validated as prognostic and/or predictive biomarkers. One of this biomarkers being the mutated IDH1 gene, which correlates with better prognostic in primary GBM and anaplastic astrocytoma (Yan H et al. 2009 N Engl J Med 360:765-773). However, these biomarkers have to be assayed on solid patients biopsies, which is an invasive technique and not compatible with the detection of such biomarkers along the curse of the disease.

Interestingly, the detection of molecular biomarkers from liquid biopsies is compatible with the possibility to assay them along the course of the disease, and is not limited to the solid biopsy, at the beginning of the disease. Within liquid biopsies, it is possible to detect circulating nucleic acid, metabolites, tumour cells and circulating extracellular vesicles (EV) produced by the tumour cells from the original tumour.

The EVs can be classified based on their size into apoptotic bodies (Abs) (500 nm to several µm), shedding microvesicles (SMVs) (100-1000nm) and exosomes (EXOs) (50-200 nm). All three types of EVs can be secreted into the culture media in vitro, or can cross different biological barriers into biological fluids like blood, urine, saliva, cephalorachidian fluid, amniotic fluid, bronchoalveolar wash, synovial liquid and maternal milk.

Regarding brain tumours, it has been demonstrated that is quite difficult to detect circulating free DNA, belonging to the original tumour, from peripheral blood. In the best of the case, its detection is just a proof of concept with low reproducibility and not reliable for clinic, due in part to the low amount of such a DNA in peripheral blood. However, it has been reported higher concentration of DNA, belonging to the original tumour, within the cephalorachidian fluid, from which it can be tested.

Several IDH1 mutations have been described in tumours, although just two were identified from solid biopsies (Walter WC *et al.* 2013 Jul 23;2:e109. doi: 10.1038/mtna.2013.28) or cephalorachidian fluid (de Mattos-Arruda L et al. 2015 Nature Communications 6:88839) from glioma patients. However, regarding non-invasive or minimally invasive technology, IDH1 mutated DNA have been identified from circulating DNA isolated from peripheral blood of glioma patients with dramatically low sensitivity (60%) and very low reproducibility (Blandima Boisselier et al. 2012 Neurology 79:1693-1698). Additionally, have been specifically indicated that it is not possible to detect IDH1 mutated DNA from extracellular vesicles isolated from peripheral blood of glioma patients (Walter WC *et al.* 2013 Jul 23;2:e109. doi: 10.1038/mtna.2013.28).

Therefore, it is necessary to develop new methodology to reliably identified biomarkers, based on mutated DNA sequences, using minimally invasive technology, from peripheral blood, which allow for improving diagnostic, prognostic and follow-up of the disease.

### SUMMARY OF THE INVENTION

The present invention describes a method for detecting gene mutations and/or minority alleles which comprises the analysis of extracellular vesicles in a biological sample, preferably a liquid sample of a subject, with the proviso that the liquid sample is not cerebrospinal fluid. The present invention describes the usefulness in glioma patients by the detection of mutations in the IDH1 gene but the methods of the invention can be applied to other genes and diseases. The use of the methods of the present invention on DNA sequences within EVs isolated from peripheral blood, might be also applied for the detection of any other gene sequence carrying point mutations, small deletions or amplifications, and fusion genes with clinical or biological value. This technology is especially valuable for the evaluation of biomarkers in pathologies where solid pathological sample is not available or minimally invasive approaches are neccesary. These pathologies include but are not limited to inoperable tumours, inaccesible tumours or those for which biopsies or surgical removal of cancerous tissue is too risky.

In a first aspect the present invention refers to a method for detecting a gene mutation and/or a minority allele with a ratio below at least 1%, preferably below at least 0.1%, for example 10⁻³ to 10⁻⁶ mutant to wild-type DNA, compared to the wild type gene or allele comprising:
a) extracting a nucleic acid and/or a protein from the (total) extracellular vesicles (preferably total extracellular vesicles) isolated from a biological sample of a subject, with the proviso that the biological sample is not cerebrospinal fluid,
b) enriching of the mutation and/or the minority allele, and
c) identifying the gene mutation and/or the minority allele.

From now on, the "first method" of the invention.

The extraction of the nucleic acid and/or a protein can be done through protocols known in the state of the art.

The term nucleic acid includes genomic deoxyribonucleic acid (DNA) or DNA encoding double or single stranded, ribonucleic acid (RNA) and both sense and antisense strands.

The gene mutation and/or minority allele can be also compared to a reference sample. The term "reference samples" as understood in the present invention refers, for example, but not limited to, the samples taken from individuals who have a known molecular profile. This molecular profile can be also informative of good or bad prognosis for a certain disease.

Among the minority alleles, genetic polymorphism can be found. The term "genetic polymorphism" refers to a variation in nucleotide sequence of the strand of DNA having at least one frequency of 1% in a population of individuals. Genetic polymorphisms may be variations of one or more nucleotides. The single nucleotide polymorphism, "single nucleotide polymorphism" or SNP, generally gives rise to two different alleles. Polymorphisms or SNPs may contain information on the variation of a single polymorphism or haplotype information if they are SNP labels or "tag-SNP". In the present invention the term "polymorphism" and "SNP" are used interchangeably. In the present invention polymorphisms can be also SNPs of a genomic region which lies within a given haplotype block.

It is understood by "haplotype" a combination of alleles at different loci of a chromosome that are transmitted together. An haplotype can be a locus, several loci, or an entire chromosome depending on the number of recombination events that have occurred between a given set of loci. The alleles transmitted from the mother form the maternal haplotype, while alleles of paternal haplotype form the paternal haplotype. It also relates to a set of polymorphisms (SNPs) that are inherited in bloc with measures of linkage disequilibrium r2≥0,90 and identified by genotyping of the polymorphisms included in said haplotype.

Polymorphisms in a haplotype block are in linkage disequilibrium. "Linkage disequilibrium" (LD) is the property of some loci do not segregate independently, i.e., have a lower recombination frequency of 50%.

In the present invention the term "minor allele" means the allele is underrepresented in the population, as is known to the skilled person and therefore, the term "major allele" refers to the most frequent allele in the population.

The term "extracellular vesicles" or EVs as used herein refers to membrane surrounded structures released by cells, for example, tumor cells, to the medium in an evolutionally conserved manner. Examples of EVs include, without limiting to, apoptotic bodies (Abs) (500 nm to several µm), shedding microvesicles (SMVs) (100-1000nm), exosomes (EXOs) (50-200 nm), microvesicles and ectosomes. Thus, in a preferred embodiment of the first aspect of the invention, the extracellular vesicles are selected from the group consisting of Abs, SMVs, EXOs and combinations thereof. The extracellular vesicles used in step a) of the first aspect the present invention can be the total extracellular vesicles or a fraction thereof. In the context of the present invention "total extracellular vesicles" refers to the whole amount of extracellular vehicles isolated from the biological sample, while "a fraction of extracellular vesicles" refers to a part of the whole amount of total extracellular vehicles. For example, the extracellular vesicles isolated from a biological sample can be divided in three fractions depending on the size of the vesicles: The AB fraction ranged from 2000 to 500 nm, the SMV fraction showed an average size of 600 nm, and the EXO fraction displayed a size above 180 nm.

The term "biological sample" herein refers to any sample to determine the mutation and/or the minority allele who has obtained the sample, and includes, but not limiting, biological fluids or tissues of an individual, obtained by any known method one skilled in the art that serve for this purpose.

In a preferred embodiment of the first aspect of the invention the biological sample is a liquid sample and is selected from the group consisting of serum, plasma, blood, saliva, bronchoalveolar lavage fluid, milk, synovial fluid, amniotic fluid, lympha, and urine. Preferably is serum.

In a more preferred embodiment the subject suffers from a disease, preferably cancer, more preferably selected from the group consisting of: central nervous system cancer, lung, breast, prostate, melanoma, kidney, hepatic, pancreatic, colon, cervical, bladder, esophageal, head and neck cancer, sarcoma, lymphoma, leukemia, myeloma, malignant plasma cell disorder, meningioma, diffuse intrinsic pontine gliomas (DIPG) neuroblastoma and/or medulloblastoma. Preferably the central nervous system cancer is glioma and more preferably is selected from the group consisting of astrocytoma (pilocytic, diffuse, anaplastic or glioblastoma), oligodendroglyoma and oligoastrocytoma.

The term cancer (or tumor) refers herein to the disease that is associated with the presence of cells possessing characteristics known to the skilled artisan as tumor cells, such as uncontrolled proliferation, immortality, angiogenesis, resistance to programmed cell death and metastatic potential (Hanahan D et al. 2011 cell 144 (5): 646-74). In the present invention the term "cancer" includes primary tumors (with the same source of cells where they belong to) or metastatic (with cells of different origin from the place where they belong to) and also includes tumors in any degree affectation or stadium.

Detection and/or quantification of the mutation and/or minority allele of the invention may be performed by any technique known to the skilled person, for example by techniques that comprise sequencing, for genotyping, and/or amplification by polymerase chain reaction (PCR). The detection can be accomplished by using primers and/or probes.

The term "sequencing" as used herein, refers to the determination of the nucleotide of a template nucleic acid and its order.

The conditions under which the sequencing is performed generally include (a) contacting a template nucleic acid with a polymerase in a mixture further comprising a primer, a divalent cation (e.g. Mg2+), and nucleotides, generally, dNTPs and at least one ddNTP (dideoxynucleotide triphosphate), and (b) subjecting said mixture to a temperature sufficient to initiate the polymerase incorporating the nucleotides to the primer by base complementarity with the template nucleic acid, and in a population of complementary DNA molecules of different sizes. Separating said population of complementary DNA molecules, usually by electrophoresis, to determine the nucleotide sequence.

The term "amplifying", as used herein, refers to increasing the number of copies of a template nucleic acid.

The conditions under which the amplification is carried out generally include (a) contacting a template nucleic acid with a polymerase in a mixture further comprising at least one primer (being normally two primers), a divalent cation (e.g. Mg2+) and nucleotides generally dNTPs (b) subjecting said mixture to a temperature sufficient to initiate the polymerase incorporating the nucleotides to the primer by base complementarity with the template nucleic acid, and in a population of molecules complementary DNA generally the same size.

The term "template nucleic acid" or "mold", as used herein, refers to a nucleic acid molecule single-stranded or double-stranded to be amplified or sequenced.

The term "primer" (also called "oligo"), as used herein, refers to an oligonucleotide capable of acting as a point of initiation of DNA synthesis when annealed to the template nucleic acid. Preferably, the primer is an oligonucleotide of deoxyribose. Primers can be prepared by any suitable method, including for example, but not limited to, cloning and restriction of appropriate sequences and direct chemical synthesis. Primers can be designed to hybridize with specific sequences of nucleotides in the template nucleic acid (specific primers) or may be synthesized randomly (arbitrary primers).

According to the present invention a "primer" may be marked or labeled by techniques well known in the state of the art. Detectable labels include, for example, radioactive isotopes, fluorescent labels, chemiluminescent labels, bioluminescent labels and enzyme labels.

In a more preferred embodiment of the first aspect of the invention the method for the enrichment of the minority allele and/or the mutation is selected from the group consisting of: COLD-PCR, coamplification at lower denaturation temperature Polimerase Chain Reaction; ARMS, amplification refractory mutation system; ASPCR, allele-specific enzymatic amplification; ASA, allele-specific amplification; PASA, PCR amplification of specific alleles; PAMSA, PCR amplification of multiple specific alleles; COP, competitive oligonucleotide priming; E-PCR, enriched PCR; ME-PCR, mutant-enriched PCR; MAMA, mismatch amplification mutation assay; MASA, mutant allele-specific amplification; aQRT-PCR, antiprimer quenching-based real-time PCR; REMS-PCR, restriction endonuclease-mediated selective PCR; AIRS, artificial introduction of a restriction site; PNA, peptide nucleic acid; LNA, locked nucleic acid; WTB-PCR, wild-type blocking PCR; FLAG, fluorescent amplicon generation; RSM-PCR, restriction site mutation PCR; APRIL-ATM, amplification via primer ligation, at the mutation; PAP, pyrophosphate-activated polymerization; RMC, random mutation capture; CCM, chemical cleavage of mismatches; endo, endonuclease; TDG, thymine DNA glycosylase; CEL I, celery extract I; HRM, high-resolution melting; SSCP, single-strand conformation polymorphism; DGGE, denaturing gradient gel electrophoresis; CDCE, constant denaturing capillary electrophoresis; dHPLC, denaturing HPLC; and iFLP, inverse PCR-based amplified RFLP.

Another preferred embodiment of the first aspect of the invention is the method wherein the gene mutation is detected in the gene selected from the group consisting of: *IDH1, EGFR, BRAFT, KRAS, NRAS, MET, ALK, C-KIT, DDR2, ER882, ERBB3, ERBB4, FGFR2, IDH2, PALB2 PDGFR, PIK3CA, PTCH1, SMO* and *Histone H3* gene.

IDH1 refers to Isocitrate Dehydrogenase 1 (NADP+) (uc002vcu.5, identifier from the Genome Browser from the Genomics Institute at the University of California Santa Cruz). Also known as IDCD, IDH, IDHC_HUMAN, IDP, IDPC, isocitrate dehydrogenase 1 (NADP+) soluble, isocitrate dehydrogenase [NADP] cytoplasmic, NADP-dependent isocitrate dehydrogenase cytosolic, NADP-dependent isocitrate dehydrogenase peroxisomal, NADP(+)-specific ICDH, oxalosuccinate decarboxylase and PICD. SEQ ID NO: 1 refers to the *IDH1* DNA sequence, SEQ ID NO: 2 refers to its mRNA and SEQ ID NO: 3 refers to the IDH1 protein.

EGFR refers to epidermal growth factor receptor, (uc003tqk.4). Also known as: cell growth inhibiting protein 40, cell proliferation-inducing protein 61, ERBB, ERBB1, erb-b2 receptor tyrosine kinase 1, HER1, mENA, NISBD2, PIG61, proto-oncogene c-ErbB-1, and receptor tyrosine-protein kinase erbB-1.

BRAF refers to B-Raf proto-oncogene, serine/threonine kinase, (uc003vwc.5). Also known as: •94 kDa B-raf protein, BRAF1, B-raf 1, BRAF1_HUMAN, B-Raf proto-oncogene serine/threonine-protein kinase, Murine sarcoma viral (v-raf) oncogene homolog B1, p94, RAFB1, and v-raf murine sarcoma viral oncogene homolog B.

KRAS refers to Kirsten rat sarcoma viral oncogene homolog (uc001 rgp.3). Also known as: •cellular c-Ki-ras2 proto-oncogene, c-Kirsten-ras protein, C-K-RAS, c-K-ras2 protein, c-K-ras protein, KI-RAS, Kirsten rat sarcoma-2 viral (v-Ki-ras2) oncogene homolog, KRAS1, KRAS2, K-ras p21 protein, NS3, PR310 c-K-ras oncogene, RASK2, RASK_HUMAN, transforming protein p21, v-Ki-ras2 Kirsten rat sarcoma 2 viral oncogene homolog, and v-Ki-ras2 Kirsten rat sarcoma viral oncogene homolog.

NRAS refers to neuroblastoma RAS viral (v-ras) oncogene homolog (uc009wgu.4). Also known as: GTPase NRas, GTPase NRas precursor, N-ras, NRAS1, N-ras protein part 4, NS6, RASN_HUMAN, transforming protein N-Ras, and v-ras neuroblastoma RAS viral oncogene homolog.

MET refers to MET proto-oncogene, receptor tyrosine kinase (uc003vij.4). Also known as: AUTS9, c-Met, DFNB97, HGFR, and RCCP2.

ALK refers to anaplastic lymphoma receptor tyrosine kinase (uc002rmy.4). Also known as: ALK tyrosine kinase receptor, anaplastic lymphoma kinase, CD246, CD246 antigen, and NBLST3.

C-KIT refers to v-kit Hardy-Zuckerman 4 feline sarcoma viral oncogene homolog (uc010igr.4). Also known as: CD117, C-Kit, KIT_HUMAN, mast/stem cell growth factor receptor Kit, p145 c-kit, PBT, piebald trait protein, proto-oncogene c-Kit, proto-oncogene tyrosine-protein kinase Kit, SCFR, tyrosine-protein kinase Kit, and v-kit Hardy-Zuckerman 4 feline sarcoma viral oncogene-like protein.

DDR2 refers to discoidin domain receptor tyrosine kinase 2 (uc001gch.2). Also known as: MIG20a, NTRKR3, TKT, and TYRO10.

ERBB2 refers to erb-b2 receptor tyrosine kinase 2 (uc002hso.4). Also known as: CD340, HER2, HER-2, HER-2/neu, MLN 19, NEU, NGL and TKR1, ERBB3 refers to erb-b2 receptor tyrosine kinase 3 (uc001sjh.4). Also known as: c-erbB3, c-erbB-3, ErbB-3, erbB3-S, HER3, LCCS2, MDA-BF-1, p45-sErbB3, p85-sErbB3, and p180-ErbB3.

ERBB4 refers to erb-b2 receptor tyrosine kinase 4 (uc061rzr.1). Also known as: ALS19, HER4, and p180erbB4.

FGFR2 refers to fibroblast growth factor receptor 2 (uc057wle.1). Also known as: bacteria-expressed kinase, BEK, BEK fibroblast growth factor receptor, BEK protein tyrosine kinase, BFR-1, CD332, CEK3, CFD1, ECT1, FGFR2_HUMAN, FGF receptor, keratinocyte growth factor receptor, KGFR, K-SAM, protein tyrosine kinase, receptor like 14, TK14, TK25, and tyrosylprotein kinase.

IDH2 refers to isocitrate dehydrogenase 2 (NADP+), mitochondrial (uc002box.4). Also known as: D2HGA2, ICD-M, IDH, IDHM, IDHP_HUMAN, IDP, IDPM, isocitrate dehydrogenase [NADP], mitocondrial, mNADP-IDH, NADP(+)-specific ICDH, and oxalosuccinate decarboxylase.

PALB2 refers to partner and localizer of BRCA2 (uc002dlx.2). Also known as: FANCN and PNCA3.

PDGFR refers to platelet-derived growth factor receptor, beta polypeptide (uc003han.5). Also known as: beta-type platelet-derived growth factor receptor, CD140 antigen-like family member B, CD140B, PDGFR1, PDGFR-1, PDGFR-beta, PDGF-R-beta, PGFRB_HUMAN, platelet-derived growth factor receptor 1, and platelet-derived growth factor receptor beta.

PIK3CA refers to phosphatidylinositol-4,5-bisphosphate 3-kinase catalytic subunit alpha (uc003fjk.4). Also known as: p110-alpha, phosphatidylinositol 3-kinase, catalytic, 110-KD, alpha, phosphatidylinositol 3-kinase, catalytic, alpha polypeptide, phosphatidylinositol-4,5-bisphosphate 3-kinase 110 kDa catalytic subunit alpha, phosphatidylinositol-4,5-bisphosphate 3-kinase, catalytic subunit alpha, phosphatidylinositol 4,5-bisphosphate 3-kinase catalytic subunit alpha isoform, phosphoinositide-3-kinase, catalytic, alpha polypeptide, PI3K, PI3K-alpha, PI3-kinase p110 subunit alpha, PK3CA_HUMAN, ptdIns-3-kinase subunit p110-alpha, and serine/threonine protein kinase PIK3CA.

PTCH1 refers to patched 1 (uc004avk.5). Also known as: BCNS, FLJ26746, FLJ42602, HPE7, NBCCS, patched, patched homolog 1 (Drosophila), PTC, PTC1, PTC1_HUMAN, and PTCH.

SMO refers to Homo sapiens smoothened, frizzled class receptor (uc003vor.4). Also known as: A4D1K5, NM_005631.4, NP_005622.1, Q99835, SMOH, SMO_HUMAN, uc003vor.3

Histone H3 gene refers to Homo sapiens histone cluster 3, H3 (HIST3H3) (uc001 hsx.1). Also known as: B2R5K3, H31T_HUMAN, H3FT, NM_003493.2, NP_003484.1, Q16695, Q6FGU4.

In another preferred embodiment of the first aspect of the invention the gene is the *IDH1* gene. With the methods of the invention any mutation in the *IDH1* gene can be detected.

In a more preferred embodiment, when the mutation in the *IDH1* gene is detected, it is selected at least in the position 6745, 6746 or 6747, which correspond in the messenger RNA to positions 394, 395 and 396 respectively, (which result in change in position 132 in the protein IDH1) and combinations thereof. Therefore, in a preferred embodiment the mutation in the *IDH1* gene results in a mutation in the IDH1 protein in position 132. In a more preferred embodiment the mutation in the *IDH1* gene results in a mutation in the IDH1 protein selected from R132H, R132C, R132S, R132G or R132L.

Another preferred embodiment of the first aspect of the invention refers to the method wherein in the step (b) the enrichment is permormed using the pair of primers SEQ ID NO: 4 (5'-CGGTCTTCAGAGAAGCCATT-3') and SEQ ID NO: 5 (5'-GCAAAATCACATTATTGCCAAC-3'), or the pair of primers SEQ ID NO: 6 (5'- GTTGAAACAAATGTGGAAATC - 3') and SEQ ID NO: 7 (5'-TAATGGGTGTAGATACCAAAAG- 3').

Another preferred embodiment of the first aspect of the invention refers to the method wherein the gene mutation is detected in *IDH1* gene, it is detected by Cold-PCR comprising the following steps:
a. a denaturation step of 90-99°C (90, 91, 92, 93, 94, 95, 96, 97, 98 or 99 °C), preferably 96°C, during 5 to 15 minutes (5, 6, 7, 8, 9, 10, 11, 12, 13, 14 or 15 minutes), preferably 10 minutes;
b. a following step of 15 to 35 cycles (15, 16, 17, 18, 19, 20, 21, 22, 23, 24, 25, 26, 27, 28, 29, 30, 31, 32, 33, 34 or 35 cycles), preferably 20 cycles, comprising a denaturation step of 90-98°C (90, 91, 92, 93, 94, 95, 96, 97 or 98 °C), preferably 96°C, during 10-20 seconds (10, 11, 12, 13, 14, 15, 16, 17, 18, 19 or 20 seconds), preferably 15 seconds; and an annealing and primer extension step of 55-65°C (55, 56, 57, 58, 59, 60, 61, 62, 63, 64 or 65 °C), preferably 60°C, during 10-20 seconds (10, 11, 12, 13, 14, 15, 16, 17, 18, 19 or 20 seconds), preferably 15 seconds;
c. a next step of 15 to 35 cycles (15, 16, 17, 18, 19, 20, 21, 22, 23, 24, 25, 26, 27, 28, 29, 30, 31, 32, 33, 34 or 35 cycles), preferably 30 cycles, comprising a denaturation step (critical temperature) of 70-85°C (70, 71, 72, 73, 74, 75, 76, 77, 78, 79, 80, 81, 82, 83, 84 or 85 °C), preferably 79 °C, during 10-20 seconds (10, 11, 12, 13, 14, 15, 16, 17, 18, 19 or 20 seconds), preferably 15 seconds; and an annealing and primer extension step of 55-65°C, preferably 60°C, during 10-20 seconds (10, 11, 12, 13, 14, 15, 16, 17, 18, 19 or 20 seconds), preferably 15 seconds; and
d. a final step wherein the amplified DNA is maintained at a temperature below 20°C, preferably at 0, 1, 2, 3, 4, 5, 6, 7, 8, 9, 10, 11, 12, 13, 14, 15, 16, 17, 18, 19 or 20 °C, preferably during the time estimated by the expert, more preferably up to the use of the amplicon.

Therefore a more preferred embodiment of the first aspect of the invention refers to the method wherein the gene mutation is detected in *IDH1* gene, it is detected by Cold-PCR comprising the following steps:
a. a denaturation step of 96°C, during 10 minutes;
b. a following step of 20 cycles, comprising a denaturation step of 96°C, during 15 seconds; and an annealing and primer extension step of 60°C, during 15 seconds;
c. a next step of 30 cycles, comprising a denaturation step (critical temperature) of 79°C, during 15 seconds; and an annealing and primer extension step of 60°C, during 15 seconds; and
d. a final step wherein the amplified DNA is maintained at a temperature below 20°C, preferably at 0, 1, 2, 3, 4, 5, 6, 7, 8, 9, 10, 11, 12, 13, 14, 15, 16, 17, 18, 19 or 20 °C, preferably during the time estimated by the expert, more preferably up to the use of the amplicon.

As the skilled person in the art understands, if different primers and/or probes related to different genes are used simultaneously in the method of the first aspect of the invention for the enrichment of the mutations or alleles, multiple genes mutations and/or minority alleles can be detected. Thus, the present invention also encompasses the combination of pair of primers related to the same or different genes, wherein the genes comprise at least 2, 3, 4, 5, 6, 7, 8, 9, 10, 11, 12, 13, 14, 15, 16, 17, 18, 19 o 20 genes of the list of genes selected from *IDH1, EGFR, BRAFT, KRAS, NRAS, MET, ALK, C-KIT, DDR2, ERBB2, ERBB3, ERBB4, FGFR2, IDH2, PALB2, PDGFR, PIK3CA, PTCH1, SMO* and *Histone H3* gene.

A second aspect of the present invention refers to an *in vitro* method for the prognosis and/or the diagnosis of a disease associated with a gene mutation and/or a minority allele with a ration below at least 1%, more preferably wherein the mutant to wild-type DNA is10⁻³ to 10⁻⁶, in a subject comprising detecting the presence of the mutation and/or the minority allele associated with the disease by the method of the first aspect of the present invention, wherein the detection of said mutation and/or the minority allele is indicative that the subject suffers from or is going to suffer from said disease or the detection of said mutation is indicative of good or bad prognosis of said disease. From now on, the "second aspect" of the invention.

Therefore the second aspect of the invention comprises extracting a nucleic acid and/or a protein from the extracellular vesicles isolated from a biological sample of a subject, preferably a liquid sample, with the proviso that the biological sample is not cerebrospinal fluid. A preferred embodiment comprises also enriching of the mutation and/or the minority allele, and identifying the gene mutation and/or the minority allele.

The term "Extracellular vesicles" has been defined in previous paragraphs. In a preferred embodiment of the second aspect of the invention, the extracellular vesicles are selected from the group consisting of Abs, SMVs, EXOs and combinations thereof. In another preferred embodiment, the extracellular vesicles obtained from the biological sample are the total extracellular vesicles or a fraction of the extracellular vesicles.

In a preferred embodiment of the second aspect of the invention the biological sample, preferably liquid sample, is selected from the group consisting of serum, plasma, blood, saliva, bronchoalveolar lavage fluid, milk, synovial fluid, amniotic fluid, lympha and urine. Preferably is serum.

In a more preferred embodiment of the second aspect of the invention the method for the enrichment of the minority allele and/or the mutation is selected from the group consisting of: COLD-PCR, coamplification at lower denaturation temperature Polimerase Chain Reaction; ARMS, amplification refractory mutation system; ASPCR, allele-specific enzymatic amplification; ASA, allele-specific amplification; PASA, PCR amplification of specific alleles; PAMSA, PCR amplification of multiple specific alleles; COP, competitive oligonucleotide priming; E-PCR, enriched PCR; ME-PCR, mutant-enriched PCR; MAMA, mismatch amplification mutation assay; MASA, mutant allele-specific amplification; aQRT-PCR, antiprimer quenching-based real-time PCR; REMS-PCR, restriction endonuclease-mediated selective PCR; AIRS, artificial introduction of a restriction site; PNA, peptide nucleic acid; LNA, locked nucleic acid; WTB-PCR, wild-type blocking PCR; FLAG, fluorescent amplicon generation; RSM-PCR, restriction site mutation PCR; APRIL-ATM, amplification via primer ligation, at the mutation; PAP, pyrophosphate-activated polymerization; RMC, random mutation capture; CCM, chemical cleavage of mismatches; endo, endonuclease; TDG, thymine DNA glycosylase; CEL I, celery extract I; HRM, high-resolution melting; SSCP, single-strand conformation polymorphism; DGGE, denaturing gradient gel electrophoresis; CDCE, constant denaturing capillary electrophoresis; dHPLC, denaturing HPLC; and iFLP, inverse PCR-based amplified RFLP.

Another preferred embodiment of the second aspect of the invention is the method wherein the gene mutation is detected in the gene selected from the group consisting of: IDH1, EGFR, BRAFT, KRAS, NRAS, MET, ALK, C-KIT, DDR2, ERBB2, ERBB3, ERBB4, FGFR2, IDH2, PALB2 PDGFR, PIK3CA, PTCH1, SMO and Histone H3 gene.

As explained for the first aspect of the invention, multiple gene mutations or alleles can be detected if more than one pair of primers is used. Thus, the second aspect of the invention also encompasses the combination of pair of primers related to the same or different genes, wherein the genes comprise at least 2, 3, 4, 5, 6, 7, 8, 9, 10, 11, 12, 13, 14, 15, 16, 17, 18, 19 o 20 genes of the list of genes selected from *IDH1, EGFR, BRAFT, KRAS, NRAS, MET, ALK, C-KIT, DDR2, ERBB2*, *ERBB3, ERBB4, FGFR2, IDH2, PALB2, PDGFR, PIK3CA, PTCH1, SMO* and *Histone H3* gene.

In another preferred embodiment of the second aspect of the invention the gene is the *IDH1* gene. With the methods of the invention any mutation in the *IDH1* gene can be detected.

In a more preferred embodiment of the second aspect, when the mutation in the *IDH1* gene is detected, it is selected at least in the position 6745, 6746 or 6747, which correspond in the messenger RNA to positions 394, 395 and 396 respectively, (which result in change in position 132 in the protein IDH1) and combinations thereof. Therefore, in a preferred embodiment the mutation in the *IDH1* gene results in a mutation in the IDH1 protein in position 132. In a more preferred embodiment the mutation in the *IDH1* gene results in a mutation in the IDH1 protein selected from R132H, R132C, R132S, R132G or R132L.

Another preferred embodiment of the second aspect of the invention refers to the method wherein in the step (b) the enrichment is performed using the pair of primers SEQ ID NO: 4 (5'-CGGTCTTCAGAGAAGCCATT-3') and SEQ ID NO: 5 (5'-GCAAAATCACATTATTGCCAAC-3'), or the pair of primers SEQ ID NO: 6 (5'- GTTGAAACAAATGTGGAAATC - 3') and SEQ ID NO: 7 (5'-TAATGGGTGTAGATACCAAAAG- 3').

Another preferred embodiment of the second aspect of the invention refers to the method wherein the gene mutation is detected in *IDH1* gene, it is detected by Cold-PCR comprising the following steps:
a. a denaturation step of 90-99°C (90, 91, 92, 93, 94, 95, 96, 97, 98 or 99 °C), preferably 96°C, during 5 to 15 minutes (5, 6, 7, 8, 9, 10, 11, 12, 13, 14 or 15 minutes), preferably 10 minutes;
b. a following step of 15 to 35 cycles (15, 16, 17, 18, 19, 20, 21, 22, 23, 24, 25, 26, 27, 28, 29, 30, 31, 32, 33, 34 or 35 cycles), preferably 20 cycles, comprising a denaturation step of 90-98°C (90, 91, 92, 93, 94, 95, 96, 97 or 98 °C), preferably 96°C, during 10-20 seconds (10, 11, 12, 13, 14, 15, 16, 17, 18, 19 or 20 seconds), preferably 15 seconds; and an annealing and primer extension step of 55-65°C (55, 56, 57, 58, 59, 60, 61, 62, 63, 64 or 65 °C), preferably 60°C, during 10-20 seconds (10, 11, 12, 13, 14, 15, 16, 17, 18, 19 or 20 seconds), preferably 15 seconds;
c. a next step of 15 to 35 cycles (15, 16, 17, 18, 19, 20, 21, 22, 23, 24, 25, 26, 27, 28, 29, 30, 31, 32, 33, 34 or 35 cycles), preferably 30 cycles, comprising a denaturation step (critical temperature) of 70-85°C (70, 71, 72, 73, 74, 75, 76, 77, 78, 79, 80, 81, 82, 83, 84 or 85 °C), preferably 79 °C, during 10-20 seconds (10, 11, 12, 13, 14, 15, 16, 17, 18, 19 or 20 seconds), preferably 15 seconds; and an annealing and primer extension step of 55-65°C, preferably 60°C, during 10-20 seconds (10, 11, 12, 13, 14, 15, 16, 17, 18, 19 or 20 seconds), preferably 15 seconds; and
d. a final step wherein the amplified DNA is maintained at a temperature below 20°C, preferably at 0, 1, 2, 3, 4, 5, 6, 7, 8, 9, 10, 11, 12, 13, 14, 15, 16, 17, 18, 19 or 20 °C, preferably during the time estimated by the expert, more preferably up to the use of the amplicon.

Therefore a more preferred embodiment of the second aspect of the invention refers to the method wherein the gene mutation is detected in *IDH1* gene, it is detected by Cold-PCR comprising the following steps:
a. a denaturation step of 96°C, during 10 minutes;
b. a following step of 20 cycles, comprising a denaturation step of 96°C, during 15 seconds; and an annealing and primer extension step of 60°C, during 15 seconds;
c. a next step of 30 cycles, comprising a denaturation step (critical temperature) of 79°C, during 15 seconds; and an annealing and primer extension step of 60°C, during 15 seconds; and
d. a final step wherein the amplified DNA is maintained at a temperature below 20°C, preferably at 0, 1, 2, 3, 4, 5, 6, 7, 8, 9, 10, 11, 12, 13, 14, 15, 16, 17, 18, 19 or 20 °C, preferably during the time estimated by the expert, more preferably up to the use of the amplicon.

In a preferred embodiment of the second aspect of the invention the disease associated with a gene mutation is selected from the group consisting of:
central nervous system, lung, breast, prostate, melanoma, kidney, hepatic, pancreatic, colon, cervical, esophageal, bladder, head and neck cancer, sarcoma lymphoma, leukemia, myeloma, malignant plasma cell disorder, meningioma, diffuse intrinsic pontine gliomas (DIPG), neuroblastoma and/or medulloblastoma. Preferably is glioma.

In a more preferred embodiment of the second aspect of the present invention, wherein the cancer is glioma, it is selected from the group consisting of astrocytoma (pilocytic, diffuse, anaplastic or glioblastoma), oligodendroglyoma and oligoastrocytoma.

In a more preferred embodiment of the second aspect of the present invention, wherein the cancer is glioma, the mutation detected is in the gene IDH1, and preferably the IDH1 gene mutated encodes an IDH1 protein with a mutation in position 132, preferably R132H, R132C y/o R132G, more preferably is R132H, then in these cases the subject has good prognosis.

The term "prognosis" in the present invention refers to the ability to detect patients with a high or low probability of recurrence, for example after surgery, or the ability to determine in a patient how the disease will evolve in its severity. The term "prognosis" also refers to the ability to detect subjects already diagnosed with a disease with high likelihood of a worsening of the disease. This poor outcome or "poor prognosis" can be defined as a reduced chance of achieving remission of the disease, considering that one skilled in the art knows that there are multiple ways to define this ideal state of remission in each disease. A high probability of recurrence was associated with a bad prognosis, whereas a low probability of recurrence is associated with a good prognosis. It is understood by "recurrence" the reappearance of the disease, in this case, the cancer indicated above, more preferably a glioma. It is understood therefore as "good prognosis" contrary to what previously described, that is, more likely to reach a state of remission or low disease activity, or be able to get an adequate therapeutic response according to the criteria defined by the expert in the field or also relates to a spontaneous remission.

The term "diagnosis" in the present invention refers to the determination of which disease or condition is causing a person's signs and symptoms. Both the process of determining which disease or condition is present and the conclusion that is reached by this process are part of the "diagnosis". The foundation of diagnosis is always the information from the history, the physical examination, and in the present disclosure diagnostic methods as defined above are also used during the process. A diagnosis can include determining whether a person suffering from a particular already diagnosed condition (e.g. cancer, preferably, glioma) has one or more further complications.

The first and the second methods of the invention are *in vitro* methods. The term "in vitro" refers to the method of the invention is done outside the body of the subject.

The first and second aspect of the present invention can be applied along any stage of disease.

Any of the steps of the methods of the invention may be fully or partially automated. In addition to those specified above steps, the methods described herein may include additional steps, for example, related to the pre-treatment of the sample or with the extraction of the extracellular vesicles or the extraction of the genetic material or proteins.

The methods of the invention allow obtaining useful information for therapeutic decision-making, allowing selecting patients most likely to need intensive treatment. This results aid a lowering of the direct and indirect costs of the disease and improved quality of life for patients.

The present invention is therefore also useful to select a treatment for a subject in need thereof, therefore is useful to personalise a treatment.

The present invention also refers to a kit or device that comprises the elements necessary to perform the methods of the present invention. For example, the kit or device of the invention can comprise primers or probes. The primers and/or probes may be marked or labeled by techniques well known in the state of the art, detectable labels include, for example, radioactive isotopes, fluorescent labels, chemiluminescent labels, bioluminescent labels and enzyme labels. A preferred embodiment of the kit refers to a kit that comprises any one of the primers disclosed in SEQ ID NO: 4, SEQ ID: 5, SEQ ID NO: 6 and/or SEQ ID: 7, or any combinations thereof.

The kit of the present invention can also comprise at least a DNA polymerase a reverse transcriptase, an RNA polymerase or a fluorophore. In addition the kit may comprise a mixture of deoxynucleotide triphosphate (dNTPs), a mixture of nucleotide triphosphate (NTPs), deoxyribonuclease (DNase), inhibitors of ribonuclease (RNase), Dithiothreitol (DTT), inorganic pyrophosphatase (PPi) buffers and enzymes required to perform the methods of the invention. The kits may also contain other molecules, genes, protein or probes of interest, which serve as positive and/or negative controls.

In the present invention the probe and/or primers may be located on a solid support, for example, without limitation, glass, plastic, tubes, multiwell plates, membranes, or any other known carrier, or added at any time during the development of the detection method.

The present invention also refers to the use of the kit or device of the present invention to detect a gene mutation and/or a minority allele with a ration below at least 1%, more preferably wherein the mutant to wild-type DNA is10⁻³ to 10⁻⁶, in (total) extracellular vesicles isolated from a biological sample, preferably a liquid sample with the proviso that is not a cerebrospinal fluid sample. The term "Extracellular vesicles" has been defined in previous paragraphs. In a preferred embodiment, the extracellular vesicles are selected from the group consisting of Abs, SMVs, EXOs and combinations thereof. In another preferred embodiment, the extracellular vesicles obtained from the biological sample are the total extracellular vesicles or a fraction of the extracellular vesicles.

Also it is referred to the use of the kit or device for the prognosis and/or the diagnosis of a disease associated with a gene mutation and/or a minority allele with a ration below at least 1%, more preferably wherein the mutant to wild-type DNA is10⁻³ to 10⁻⁶, in a subject comprising detecting the presence of the mutation and/or the minority allele associated with the disease by the method of the first aspect of the present invention, wherein the detection of said mutation and/or the minority allele is indicative that the subject suffers from or is going to suffer from said disease or the detection of said mutation is indicative of good or bad prognosis of said disease. And it is also referred to the use of the kit or device to select a treatment for a subject in need thereof, therefore is useful to personalise a treatment.

It is meant by "treatment" to all means used to cure or alleviate a disease.

Another aspect of the invention relates to the use *in vitro* of the (total) extracellular vesicles of a biological sample, preferably a liquid sample, with the proviso that the liquid sample is not a cerebrospinal fluid, as biomarker for a disease, preferably cancer, more preferably glioma. More preferably as diagnostic and/or prognostic biomarkers of glioma. In a preferred embodiment, the extracellular vesicles are selected from the group consisting of Abs, SMVs, EXOs and combinations thereof. In another preferred embodiment, the extracellular vesicles obtained from the biological sample are the total extracellular vesicles or a fraction of the extracellular vesicles.

In the present invention the term "subject", "individual" and "patient" are used interchangeably.

In the present invention in the methods and uses herein the subject is preferably a human. The human can be of any age, race and gender.

In the present invention the subject can have a congenital disease that manifests itself from birth, whether caused by a disorder during embryonic development or as a result of an inherited defect. Therefore the methods of the present invention can be used to detect a congenital disease by the detection of a gene mutation and/or a minority allele in a subject.

Another also preferred realization of the first and second aspects of the present invention refers to the method where the biological sample is fresh, frozen, fixed or fixed and embedded in paraffin.

In the present invention the term "detection" also includes quantitation.

In the present invention the terms "amino acid sequence" or "protein" are used here interchangeably and refer to a polymeric form of amino acids of any length, which may or may not be, chemically or biochemically modified. The term "residue" corresponds to an amino acid.

Herein the terms "nucleic acid", "nucleotide sequence", "polynucleotide", "nucleotide sequence" and "oligonucleotide" are used interchangeably.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

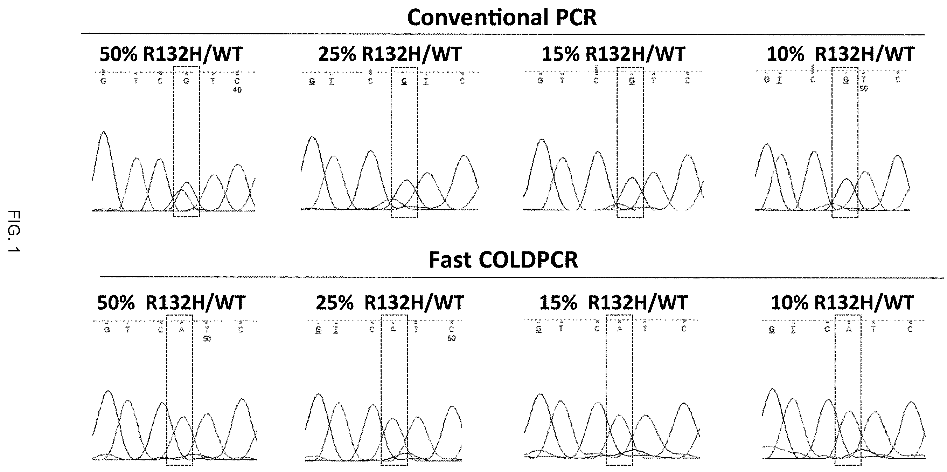

FIG. 1 Cold-PCR. It allows for the detection of IDH1 R132H mutation when the ration wild type/mutated is from few times to several orders of magnitude favorable to the wild type form. Keywords: G, dark curve; A, grey curve.

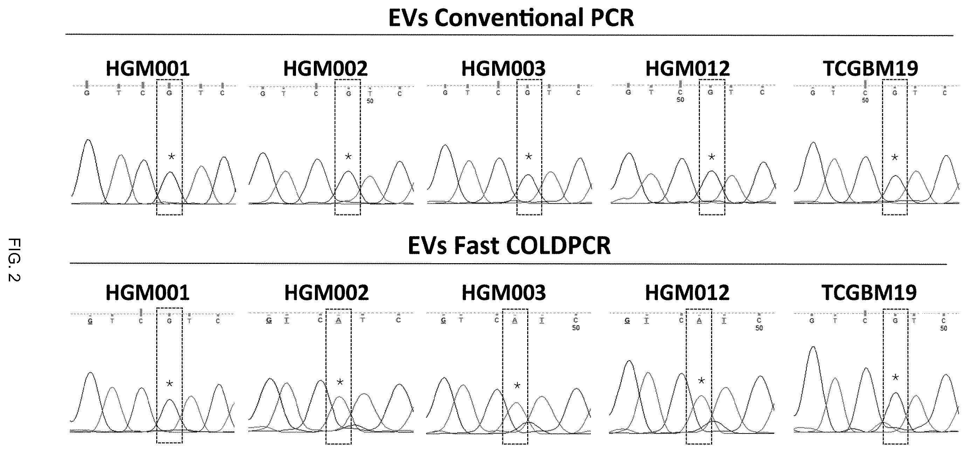

FIG. 2 The figure shows the detection of a single nucleotide mutation responsible for the IDH1^{R132H} (G□A) from solid samples by using regular PCR and their counterpart extracellular microvesicles by using Cold-PCR. Keywords: G, dark curve; A, grey curve

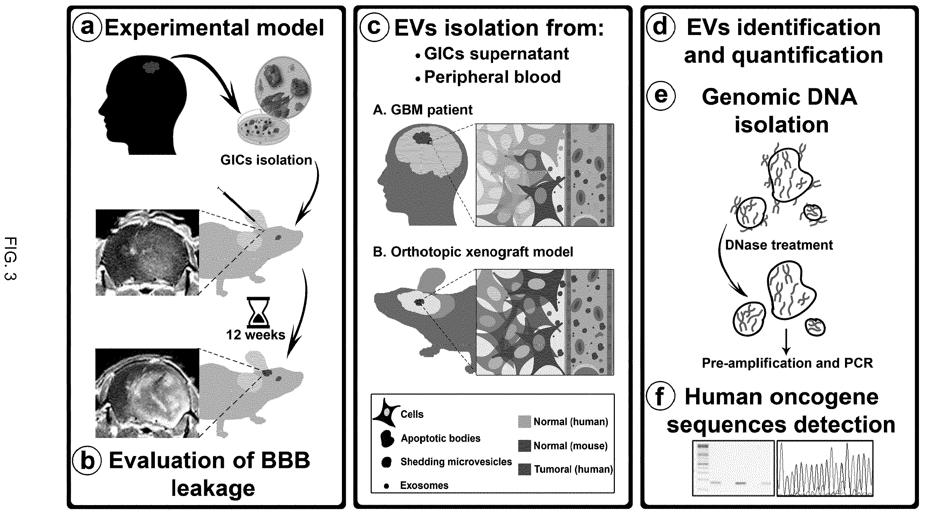

**FIG. 3****. Experimental procedure flowchart.** (**a**) Isolated hGICs from 2 GBM patients were xenotransplanted in athymic mice. After 12 weeks, the animals were transcardially perfused. (**b**) BBB permeability was evaluated using three assays: MRI, Evans Blue staining, and albumin extravasation. (**c**) EVs (ABs, SMVs, and EXOs) were isolated from hGIC-enriched culture supernatants and from mouse peripheral blood. (**d**) EVs were identified using TEM, tracking analysis, and CD63 tetraspanin quantification. (**e**) To ensure that the analysed DNA was confined within the EVs, supernatants and plasma were treated with DNase before gDNA isolation; after the isolation, gDNA was pre-amplified before performing PCR analysis with human-specific primers. (**f**) Oncogene sequences detected were sequenced to confirm their human origin.

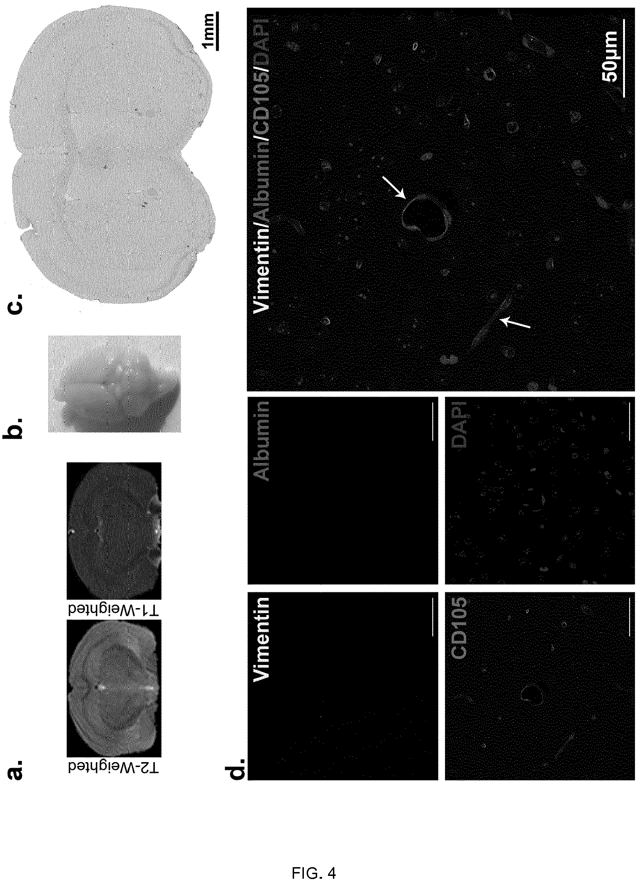

**FIG. 4****. Immunodeficient mice treated with control buffer (PBS) show an intact BBB.** (**a**) T2- and T1-weighted images of normal mouse brain injected with PBS. (**b**) No Evans Blue dye extravasation. (**c**) No human vimentin (V9) staining is detected, showing that no human cells were injected. (**d**) Immunofluorescence staining, positive for mouse CD105 (white arrows) and negative for human vimentin and mouse albumin. Nuclei were stained with DAPI.

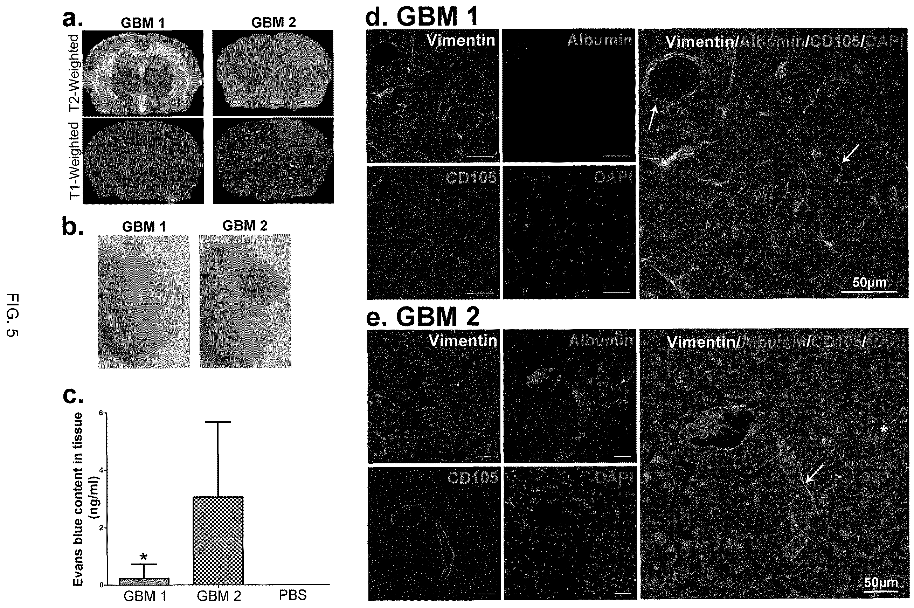

**FIG. 5****. Evaluation of BBB leakage in two GBM models: GBM1 presents an intact BBB.** (**a**) Representative T2- and T1-weighted images of GBM1 and GBM2. GBM1 features an intact BBB, as revealed by the lack of any contrast enhancement. GBM2 shows a well-defined mass and a homogeneous enhancement, which indicates that the BBB integrity is compromised. (**b**) Evans Blue extravasation. Examination of the brains of perfused animals previously stained with Evans Blue confirmed BBB disruption in the GBM2 model. (**c**) Quantification of Evans Blue extravasation. (d-**e**) Immunofluorescence staining of human vimentin, mouse CD105, and mouse albumin. Nuclei were stained with DAPI. GBM1 presents no sign of albumin staining throughout the tissue, which indicates that the BBB is intact. GBM2 features a leaky BBB, as shown by albumin spreading (white asterisk) from the blood vessels (white arrows) through the tissue.

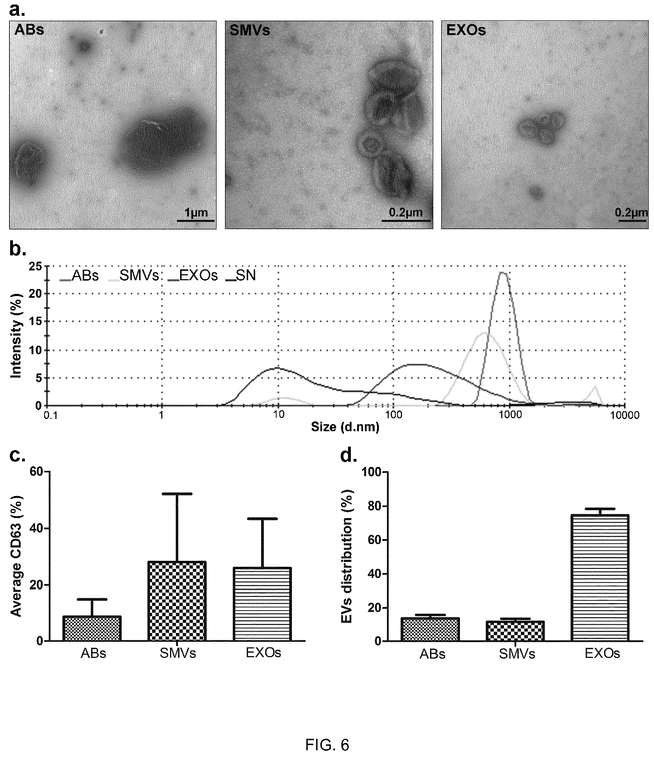

**FIG. 6****. Morphological characterisation of distinct types of EVs in hGICs from GBM1.** (**a**) Transmission electron microscopy images. ABs: apoptotic bodies (500 nm to 1 µm), SMVs: shedding microvesicles (500-150 nm); and EXOs: exosomes (150-60 nm). (**b**) Size distribution of EVs, as measured using Nanosizer tracking analysis. (**c**) Quantification of the tetraspanin cell-surface glycoprotein CD63, and (**d**) relative distribution of EVs.

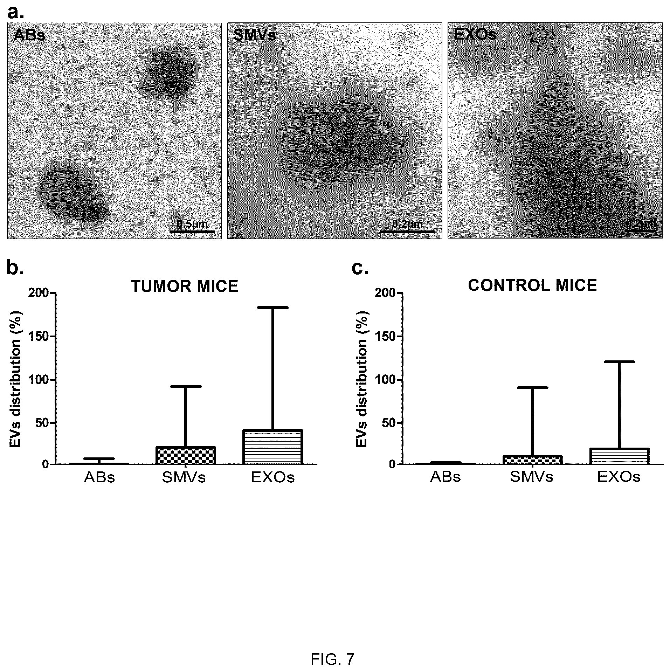

**FIG. 7****. Morphological characterisation and distribution of ABs, SMVs, and EXOs in mouse plasma.** (**a**) Transmission electron microscopy images. Relative distribution of EVs in tumour (**b**) and control (**c**) mice.

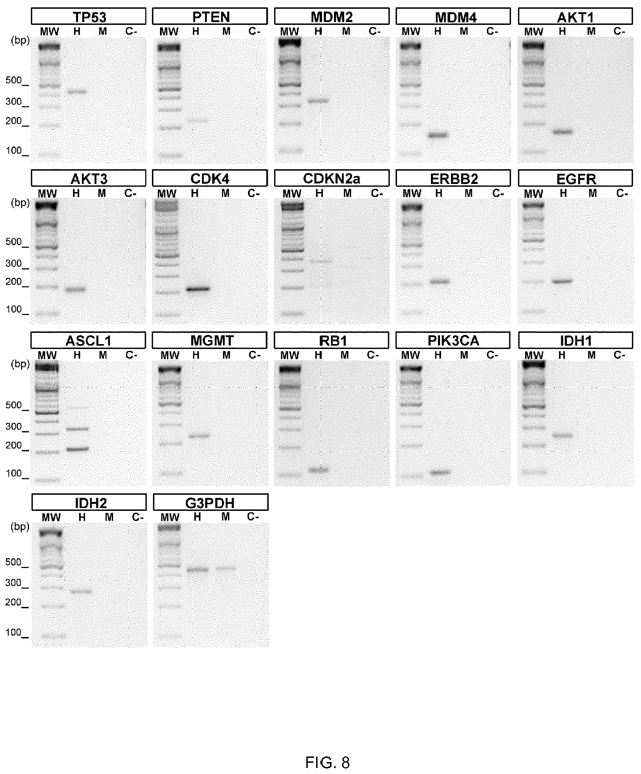

**FIG. 8****. Representative PCR product separated on a 1.8% agarose gel**; the results show that all specific primers that were designed yielded amplified sequences of the expected size from human samples (H) but not mouse samples (M). *G3PDH* was the housekeeping gene used as reference in the human and mouse samples.

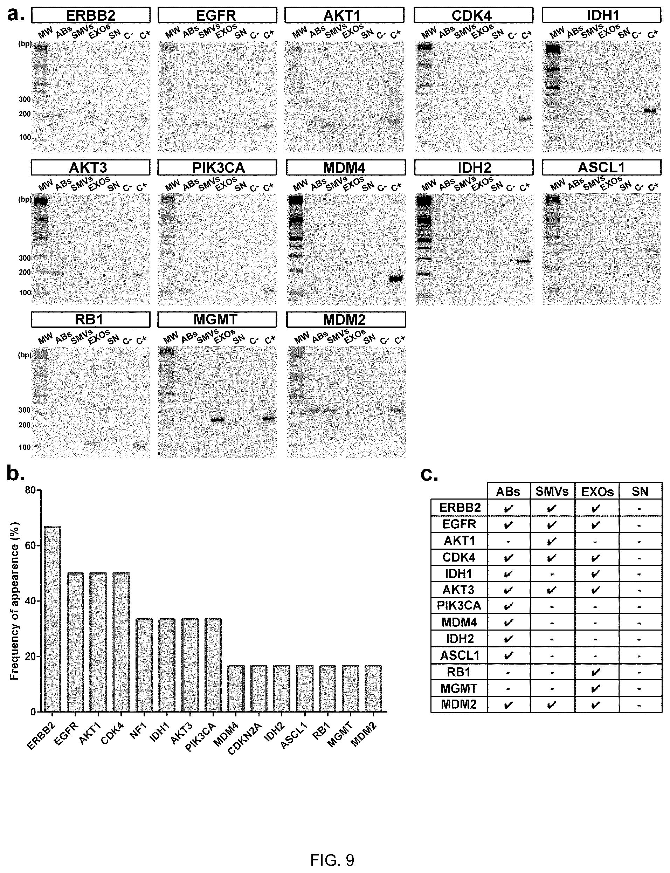

**FIG. 9****. EVs isolated from hGICs contain gDNA.** (**a**) Most representative oncogenes are present in EVs isolated from GBM1 cells. (**b**) Histogram showing the frequency of occurrence of target oncogenes. These data are the results from 6 experiments. (**c**) Presence of *ERBB2, CDK4, AKT3,* and *MDM2* sequences in all types of EVs. The remaining oncogenic sequences were found randomly in ABs, SMVs, and EXOs. No oncogene sequences were detected in the supernatant.

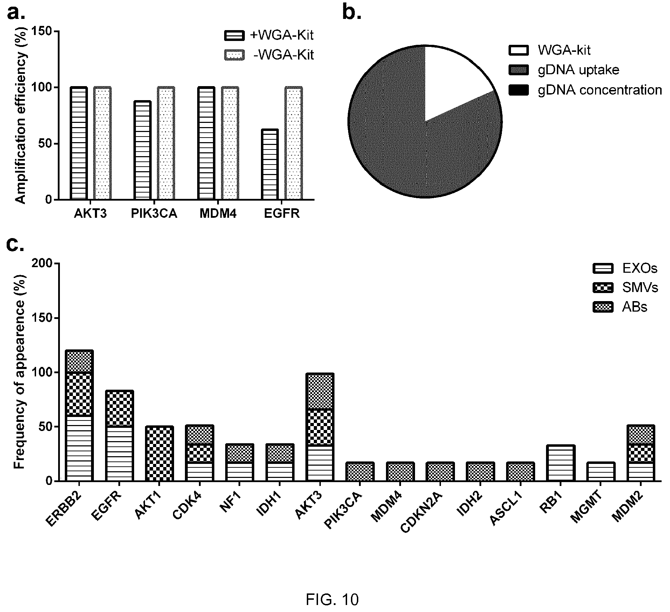

**FIG. 10****. gDNA uptake is the main cause of bias.** (**a**) Comparison of the variability induced with or without pre-amplification of 1 ng µl-1 of gDNA from hGICs, based on PCR analysis of *EGFR, AKT3, PIK3CA,* and *MDM4* transcripts. (**b**) Most of the bias observed appeared to be caused by gDNA uptake (70.4%), followed by the use of the WGA kit (15.6%). (c) Detection frequency of oncogenes in each EVs fraction.

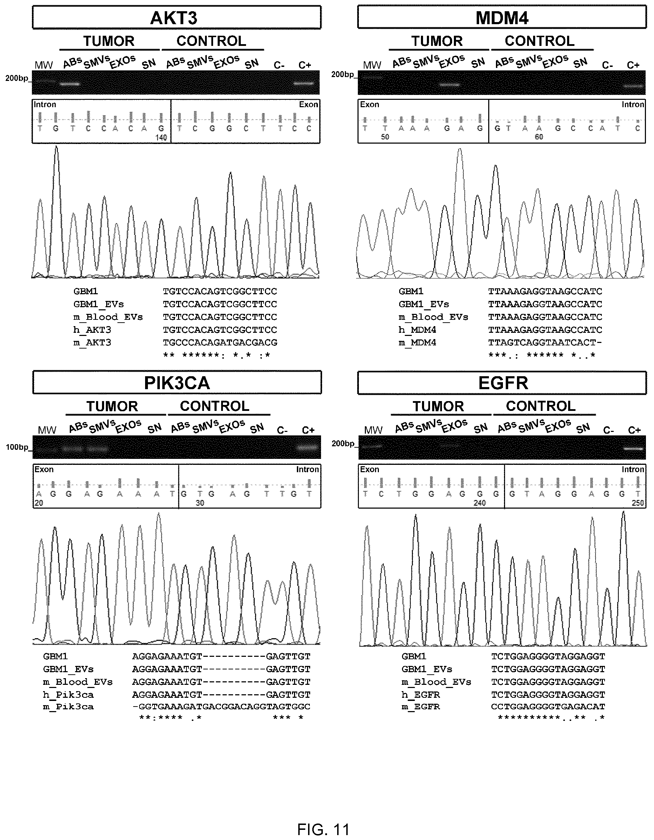

**FIG. 11****. Human gDNA sequences are confined inside EVs isolated from xenografted mice.** *AKT3, MDM4, PIK3CA,* and *EGFR* oncogene sequences were detected in EVs isolated from the peripheral blood of xenografted mice. The multiple sequence alignment shows complete homology among gDNA sequences from GBM1 hGICs, EVs isolated from hGIC-enriched culture supernatants, and EVs found in mouse peripheral blood. These results confirm the human origin of the sequences.

### EXAMPLES

### EXAMPLE 1: Procedure to detect the IDH ^{R132H} mutated DNA sequence from extracellular vesicles isolated from peripheral blood of patients by using cold PCR.

### MATERIAL AND METHODS

### Extracellular vesicle isolation from peripheral blood

Peripheral blood was collected (Within BD Vacutainer® Venous Blood Collection with gel for serum separation) from patients diagnosed with glioma at HM Hospitales (HM), Hospital General Universitario Gregorio Marañón (HGUGM) and Hospital Universitario la Fe de Valencia (HUIaFe). Permission for using this material was obtained from the ethical review board in HM, HGUGM and HUIaFe, and written informed consent was obtained from patients. Serum was first centrifuged at 1500 × g for 10 min to eliminate cell debris. Total Extracellular Vesicles (EVs) from serum samples were isolated through double ultracentrifugation at 117,000 × g for 90 min (Optima-LE 80K ultracentrifuge, 50.2 Ti rotor; Beckman Coulter). Centrifugations were performed at 4°C. Samples were then resuspended into 1 ml of phosphate buffered saline (PBS).

### Extracellular vesicle DNA isolation

Before DNA isolation, total EVs were treated with 27 Kunitz U ml-1 of DNase I (Qiagen) for 30 min at 37°C to remove potential free-DNA contaminants. Afterwards, Total DNA was extracted from EVs by using a DNeasy Blood and Tissue Kit (Qiagen) as per manufacturer recommendations.

### Cold PCR amplification of IDH1 DNA sequence

All PCRs were performed following the bellow protocol using the Paq5000 enzyme (Agilent Technologies). Pre-heated at 96°C for 10 min followed by 20 cycles of 96°C for 15 seconds (seq) and 60°C for 15 seq, and another set of 30 cycles of 79°C for 15 seq and 60°C for15 seq. Different pair of primers flanking the IDH1 R132H mutation spot can be used with similar results (for instance the forward primer 5'-CGGTCTTCAGAGAAGCCATT-3' (SEQ ID NO: 4) and reverse primer 5'-GCAAAATCACATTATTGCCAAC-3' (SEQ ID NO: 5) combination reported by Boisselier *et al.*, 2012, or the forward primer 5'-GTTGAAACAAATGTGGAAATC - 3' (SEQ ID NO: 6) and the reverse primer 5'-TAATGGGTGTAGATACCAAAAG- 3' (SEQ ID NO: 7) designed by our group. The PCR products are electrophoretically separated on 1.8% agarose gels. The band corresponding the expected size is then cut out of the gel, diluted in TE buffer and sequenced by using the ABI PRISM® BigDyeTM Terminator Cycle Sequencing Kits (Applied Biosystems) in a 48-capillary 3730 DNA Analyzer. Finally, the sequences are compared and aligned using the BLAST algorithm and CLUSTAL Omega, respectively.

**PROTOCOL OF COLD PCR**

| N° cycles | Temperature | Duration | Action |
|---|---|---|---|
| 1 | 96°C | 10 min | Denaturation |
| 20 | 96°C | 15 seg | Denaturation |
| | 60°C | 15 seg | Annealing and primer extension |
| 30 | (70-85°C) | 15 seg | Denaturation (Critical temperature, Tc) |
| | 60°C | 15 seg | Annealing and primer extension |
| 1 | 12°C | ∞ | Keeping |

Combinations of different pairs of primers flanking the IDH1 R132H mutation spot and expanding less than 300 base pair (bp) (amplicon size) can be used with similar results. For instance, we have tested the forward primer 5'-CGGTCTTCAGAGAAGCCATT-3' (SEQ ID NO: 4) and reverse primer 5'-GCAAAATCACATTATTGCCAAC-3' (SEQ ID NO: 5) combination reported by Balss *et al.*, 2008, and also different pair combinations with primers designed by our own group as shown in the table 1:

**Table 1: primers**

| **Forward Primer (5' to 3')** | **Reverse Primer (5' to 3')** | **Amplicon size** |
|---|---|---|
| CGGTCTTCAGAGAAGCCATT (SEQ ID NO: 4) | TAATGGGTGTAGATACCAAAAG (SEQ ID NO: 7) | 206 |
| CGGTCTTCAGAGAAGCCATT (SEQ ID NO: 4) | TGCATTTCTCAATTTCATACCTTGCT (SEQ ID NO: 8) | 232 |
| GTTGAAACAAATGTGGAAATC (SEQ ID NO: 6) | TGCATTTCTCAATTTCATACCTTGCT (SEQ ID NO: 8) | 288 |
| ATTCTGGGTGGCACGGTCTT (SEQ ID NO: 9) | TGCATTTCTCAATTTCATACCTTGCT (SEQ ID NO: 8) | 245 |
| ATTCTGGGTGGCACGGTCTT | GCAAAATCACATTATTGCCAAC | 142 |
| (SEQ ID NO: 9) | (SEQ ID NO: 5) | |
| ATTCTGGGTGGCACGGTCTT (SEO ID NO: 9) | TAATGGGTGTAGATACCAAAAG (SEO ID NO: 7) | 219 |

### RESULTS

In this preliminary screening samples from 29 glioma patients were analyzed (table 2). 5 solid samples (4 from HGUGM and 1 from TCGBM) were tested by immunohistochemistry. We found 2 out of 5 to be IDH1^{R132H}. All samples were also tested in our laboratory by regular PCR, from then, 4 out of 29 were positive for IDH1^{R132H}, including the 2 samples found positive by immunohistochemistry. Afterward, cold PCR were used to test 16 solid samples and 6 samples were found positive for IDH1^{R132H}, 4 previously detected by regular PCR and 2 previously negative by regular PCR. Finally, we assayed DNA isolated from extracellular vesicle isolated from peripheral blood of 5 patients, three of them positives for IDH1^{R132H} when the solid tissues were analyzed by either regular PCR or Cold-PCR. Surprisingly, the result was negative when we used regular PCR but positive when Cold PCR was utilized.

The utilization of Cold-PCR allows for the detection of IDH1 R132H mutation when the ratio IDH1^{WT} / IDH^{1R132H} is significantly favorable to the IDH1WT form. For this experiment two DNA samples were combined (Figure 1). The first sample was IDH1^{WT} / IDH1^{WT}, while the second one was IDH1^{WT} / IDH1^{R132H}. Therefore, 1/1 ratio means 75% IDH1^{WT} and 25% IDH1 ^{R132H}. Then, we carried out serial combination increasing the IDH1WT / IDH1 R132H ratio up to 1:4 where we find 90% IDH1^{WT} and 10 % IDH1^{R132H}. Under these conditions, we were able to find the single nucleotide mutation responsible for the IDH1^{R132H} (G□A) by regular PCR (upper line), when the presence of IDH1^{R132H} was above 20%. Interestingly, Cold-PCR (lower line) allowed the detection of IDH1^{R132H} (G□A) at least for concentrations as low as 10%.

The Cold-PCR technology decreases the IDH1^{wild type}/IDH1 ^{R132H} ratio, making possible the detection of the IDH1^{R132H} from extracellular microvesicles (Figura 2). The figure shows the detection of a single nucleotide mutation responsible for the IDH1^{R132H} (G□A) from solid samples by using regular PCR and their counterpart extracellular microvesicles by using Cold-PCR.

Table 2 (next page): List of samples assayed for IDH1^{wild type} and IDH1^{R132H}. Keywords: wt, IDH1^{wild type}; No, not assayed and R132H, IDH1^{R132H}.

| | | | **Solid tumor** | | | **Liquid biopsy** | |
|---|---|---|---|---|---|---|---|
| **Patients** | **Pathologic Diagnostic** | **WHO Grade** | **IHC** | **Conventional PCR** | **Fast COLDPCR** | **EVs Conventional PCR** | **EVs Fast COLDPCR** |
| **HGM001** | Metastasis | | WT | WT | WT | WT | WT |
| **HGM002** | Oligodendroglioma | II | WT | R132H | R132H | WT | R132H |
| **HGM003** | Oligodendroglioma | II | R13 2H | R132H | R132H | WT | R132H |
| **HGM012** | Oligoastrocitoma | II | R13 2H | R132H | R132H | WT | R132H |
| **TCGBM 19** | Glioblastoma | IV | WT | WT | WT | WT | WT |
| **HGM004** | Oligoastrocitoma | III | | WT | WT | - | - |
| **HGM005** | Astrocitoma | III-IV | | WT | WT | - | - |
| **HGM006** | Glioblastoma | IV | | WT | WT | - | - |
| **HGM007** | Astrocitoma | IV | | WT | WT | - | - |
| **HGM008** | Astrocitoma | III | | WT | R132H | - | - |
| **HGM009** | Glioblastoma | IV | | WT | WT | - | - |
| **HGM010** | Glioblastoma | IV | | WT | WT | - | - |
| **HGM011** | Glioblastoma | IV | | WT | WT | - | - |
| **HGM013** | Glioblastoma | IV | | WT | WT | - | - |
| **HGM014** | Glioblastoma | IV | | R132H | R132H | - | - |
| **HGM015** | Oligodendroglioma | II | | WT | - | - | - |
| **TCGBM 16** | Metastasis | | | WT | - | - | - |
| **TCGBM 18** | Glioblastoma | IV | | WT | - | - | - |
| **TCGBM 20** | Glioblastoma | IV | | WT | - | - | - |
| **TCGBM 21** | Glioblastoma | IV | | WT | - | - | - |
| **TCGBM 22** | Glioblastoma | IV | | WT | - | - | - |
| **TCGBM 23** | Glioblastoma | IV | | WT | - | - | - |
| **TCGBM 24** | Glioblastoma | IV | | WT | - | - | - |
| **TCGBM 25** | Glioblastoma | IV | | WT | - | - | - |
| **TCGBM 26** | Oligoastrocitoma anaplasico | III | | WT | - | - | - |
| **TCGBM 27** | Glioblastoma | IV | | WT | - | - | - |

| | | | | | | | |
|---|---|---|---|---|---|---|---|
| **TCGBM 28** | Glioblastoma | IV | | WT | - | - | - |
| **TCGBM 29** | Glioblastoma | IV | | WT | - | - | - |
| **TCGBM 30** | Glioblastoma | IV | | WT | R132H | - | - |

### EXAMPLE 2: The tumour-derived EVs cross intact blood brain barrier (BBB)

### MATERIAL AND METHODS

### Glioblastoma-initiating cells-enriched cultures

Two tumour samples from GBM patients (GBM1 and GBM2) were processed within 12 h after extraction as per the protocol described previously (Ayuso-Sacido, A. et al. 2008. Neurosurgery. 62(1), 223-9). Tissue samples were obtained from patients operated at Neurosurgery department, Hospital la Fe, Spain. Permission for using this material was obtained from the ethical review board in Hospital la Fe, and written informed consent was obtained from patients. Briefly, the samples were minced and washed in Ca2+/Mg2+-free HBSS (Hanks balanced salt solution). Enzymatic digestion was sequentially performed with Solution I (papain (14 U ml-1, Sigma-Aldrich) and DNase I (10 U ml-1, Sigma) in PIPES solution) for 90 min at 37°C and Solution II (papain (7 U ml-1) and DNase I (15 U ml-1) in 1:1 PIPES:proliferation medium) for 30 min at 37°C. The cells were then dissociated using diameter-tapering polished Pasteur pipettes and filtered through a 70-µm mesh, and the dissociated cells were resuspended in defined proliferative media.

### Xenotransplants

Procedures used on mice were approved by and performed according to the guidelines of the institutional animal-care committee of Principe Felipe Research Center in agreement with European Union and National directives.

Approximately 105 cells from 2 cancer-initiating cell cultures (GBM1 and GBM2) were injected into the right striatum of 10 immunosuppressed athymic nude female mice by using a stereotactic device. As negative control, PBS was injected, following the same protocol, into 2 mice. Twelve weeks after injection, mice were anaesthetised and then transcardially perfused with saline and prefixed with 4% paraformaldehyde. The brains were post-fixed for 48 h after the infusion and embedded in paraffin, after which 3-µm coronal sections were obtained.

### Magnetic resonance imaging

Magnetic resonance experiments were performed on a 7.0-T Bruker Pharmascan (Bruker Medical Gmbh, Ettlingen, Germany) superconducting magnet, with Paravision 5.1 software. T2-weighted MRI and T1-weighted MRI after paramagnetic contrastagent administration were used to assess tumour implantation and BBB integrity, at time 0 and at 90 days. Prior to scanning, mice were anaesthetised with isofluorane. T2-weighted images were acquired by using the rapid acquisition with refocused echo (RARE) sequence with the following parameters: TR/TE (repetition time/echo time) = 2500/44 ms, field of view = 2.3 cm, 6 averages, matrix size = 256 × 256, number of slices = 14, and slice thickness = 1 mm without a gap. The total scan time required to concurrently acquire T2-weighted images was 6 min.

Next, we modified certain parameters for T1-weighted images; for example, we used the multi-slice multi-echo (MSME) sequence, TR/TE = 3500/10.6 ms, 3 averages, and total time of acquisition = 3 min 2 s. The contrast agent used, 0.3 M Gd-DTPA, was injected intraperitoneally.

### Evans Blue injection and brain extraction

A 2% Evans Blue dye solution (Sigma-Aldrich) was administered (2 ml kg-1) through the tail vein, and 1 h later, the mice were transcardially perfused and the brains were extracted, weighed, and homogenised using a TissueLyser LT (Qiagen) in twice their volume of N,N-dimethylformamide (Sigma-Aldrich). The tissues were incubated overnight at 55°C and then centrifuged for 20 min at 9300 × g. The optical density (OD) of the supernatant was measured at 610-635 nm, and the amount of Evans Blue extravasation was quantified as nanograms per milligram of tissue.

### Immunofluorescence staining

Immunofluorescence staining for mouse albumin was performed to determine the vascular integrity of the brain. Brain sections obtained from GBM1 and GBM2 hGICs and PBS control xenotransplants were incubated with a 5% blocking solution of the specific serum, and then incubated (overnight, 4°C) in solutions containing these primary antibodies: goat anti-mouse CD105 (R&D Systems), goat anti-mouse albumin (Santa Cruz Biotechnology), and mouse anti-human vimentin (Santa Cruz Biotechnology). Next, Alexa Fluor-conjugated secondary antibodies were used for 1 h
(donkey anti-goat 568, rabbit anti-goat 488, and goat anti-mouse 660; Life Technologies, USA), and then nuclei were counterstained with DAPI and coverslips were mounted using FluorSave™ reagent (Millipore). Fluorescence was examined under a Leica TCS SP5 inverted confocal microscope.

### Plasma samples

Twelve weeks after the xenotransplantation, mouse peripheral blood was collected from the inferior vena cava, and centrifuged at 1500 × g for 10 min to obtain the plasma.

### Extracellular vesicles isolation

EVs from plasma samples and cell medium supernatants were isolated through differential ultracentrifugation as previously described28, with certain modifications. Briefly, to remove cellular debris, samples were centrifuged at 1200 × g for 5 min. The supernatant was carefully aspirated off without disturbing the pellet and centrifuged at 8000 × g for 20 min to obtain ABs. Next, this supernatant was centrifuged at 25,000 × g for 20 min to obtain SMVs, and then the remaining supernatant was ultracentrifuged at 117,000 × g for 90 min to obtain EXOs (Optima-LE 80K ultracentrifuge, 50.2 Ti rotor; Beckman Coulter). All centrifugations were performed at 4°C. A sample of the EV-free supernatant was collected after the ultracentrifugation and used as a negative control. Samples were immediately used or stored at -20°C.

### Transmission electron microscopy

EVs samples were individually added onto glow-discharged 150-mesh formvar copper grids (EMS™), subjected to the glow-discharge procedure (2 min, 2.4 MA), and then incubated for 2 min at 4°C. The grids were washed, negatively stained with 2% aqueous uranyl acetate solution, dried, and analysed by performing TEM (FEI Tecnai Spirit G2 and Tecnai 12) at 80 kV. EVs were classified based on their size, photographed, and counted using a Soft Image System Morada camera.

### Tracking analysis

Mean droplet sizes of EVs were measured using the dynamic light scattering method and a Zetasizer Nano ZS (Malvern Instruments, UK).

### Flow-cytometry analysis of CD63 expression

AB, SMV, or EXO samples were adsorbed onto 4-mm aldehyde/sulphate latex beads (Invitrogen, Paisley, UK) overnight at 4°C. The reaction was stopped by adding 100 mM glycine. Membrane-bound beads were washed in PBS/1% BSA, incubated with mouse anti-CD63 (Abcam, Cambridge, UK) or appropriate isotype control for 30 min at 4°C, stained with FITC-conjugated secondary antibody (R&D Systems) for 30 min at 4°C, and resuspended in 0.5 ml of PBS. Samples were analysed using a FACS Calibur flow cytometer (BD Biosciences, San Jose, CA, USA).

### DNA isolation and pre-amplification and PCR

Before DNA isolation, supernatant and plasma samples were treated with 27 Kunitz U ml-1 of DNase I (Qiagen) for 30 min at 37°C to remove potential free-DNA contaminants. Total DNA was extracted from EVs and cells by using a DNeasy Blood and Tissue Kit (Qiagen) as per manufacturer recommendations. Next, a working solution of the extracted DNA was pre-amplified using the GenomePlex Complete WGA2 Kit (Sigma-Aldrich), according to the manufacturer's instructions. All PCRs were performed following the protocol of the Paq5000 enzyme (Agilent Technologies). The final PCR products were electrophoretically separated on 1.8% agarose gels.

### Sequencing and assembly

DNA sequencing of the PCR-amplified products of AKT3, EGFR, MDM4, and PIK3CA was performed by the sequencing service at Spanish National Cancer Research Center (CNIO). Sequences were compared and aligned using the BLAST algorithm and CLUSTAL Omega, respectively.

### Statistical analyses

Statistical analyses were performed using a 2-tailed Student t test. Data are presented as means ± standard deviation and were calculated using the software package GraphPad Prism v. 5.0. Statistical values of p > 0.05 were not considered significant.

### Immunohistochemistry

Formalin-fixed paraffin-embedded sections were stained (as per the manufacturer's staining protocol) with the Bond Polymer Refine Detection Kit on a Bond-max™ fully automated staining system (Leica Microsystems GmbH, Germany), using a mouse monoclonal antibody against human vimentin (V9) (Santa Cruz Biotechnology).

### Primers design and PCR assays

Genomic sequences of known oncogenes were obtained from the UCSC Genome Browser database and primers were designed covering intron/exon boundaries by using Primer 3 software. All primers were tested in PCR with human gDNA (positive control) and mouse gDNA (negative control). All PCRs were performed following the protocol of the Paq5000 enzyme (Agilent Technologies), and final PCR products were electrophoretically separated on 1.8% agarose gels.

### Genomic DNA Microarray

Genomic DNA was quantified by spectrometry (NanoDrop ND1000, NanoDrop Technologies, Wilmington, Delaware USA). Integrity of DNA was assessed by 0.8% agarose gel electrophoresis. Non-amplification labeling of DNA (direct method) was obtained following the 'Agilent Oligonucleotide Array-Based CGH for Genomic DNA Analysis' protocol Version 4.0 (Agilent Technologies, Palo Alto, California USA. p/n G4410-90010). 500 ng of experimental and pool female reference genomic DNA samples were fragmented in a restriction digestion step. Digestion was confirmed and evaluated by DNA 7500 Bioanalyzer assay. Cyanine 3-dUTP and cyanine 5-dUTP were used for fluorescent labeling of, respectively, test and reference-digested gDNAs using the 'Agilent Genomic DNA Labeling Kit PLUS' (Agilent p/n 5188-5309) according to the manufacturer's instructions. Labeled DNA was hybridized with Human Genome CGH Microarray 44K (Agilent p/n G4426B-014950) containing 43,000+ coding and noncoding human sequences represented. Arrays were scanned in an Agilent Microarray Scanner (Agilent G2565BA) according to the manufacturer's protocol and data extracted using Agilent Feature Extraction Software 9.5.3.1 following the Agilent protocol CGH-v4_95_Feb07 ('Lowess Only' normalization correction dye bias method instead of 'Linear Only') and the QC Metric Set CGH_QCMT_Feb08 (Supplementary Fig. 5).

### Evaluation of DNA pre-amplification variability

To rule out the possibility that DNA pre-amplification procedures might be responsible for the variability observed in the PCR-amplification assays, we analysed the PCRamplification results of 4 representative DNA oncogenic sequences from hGICs (AKT3, MDM4, PIK3CAca, and EGFR) with or without a WGA kit. A representative gDNA sample from hGICs was diluted to obtain a 1 ng µl-1 working solution and was preamplified using the GenomePlex Complete WGA2 Kit (Sigma-Aldrich). The same sample was also used without the pre-amplification step. AKT3, MDM4, PIK3CA, and EGFR were amplified using conventional PCR. Cycling conditions included an initial denaturation step of 2 min at 95°C, followed by 34 cycles of 20 s at 95°C, 20 s at the primer hybridisation temperature, and 30 s at 72°C, and a final extension step at 72°C for 5 min. The experiment was performed twice to ensure reproducibility of the result. We observed a 15.6% variability when the pre-amplification step was included; AKT3 and MDM4 showed 100% reproducibility, whereas certain amount of bias was observed for PIK3CAca and EGFR. However, the absence of this step resulted in 100% reproducibility. Unexpectedly, we observed a 70.4% variability when we used the gDNA isolated from EVs as a template. These results suggest that the high variability observed does not depend on the pre-amplification procedure, and instead depends mainly on the EVs cargo (Figure 10).

### RESULTS

To evaluate the capacity of EVs of tumour cells to cross the BBB into the bloodstream, we used an orthotopic xenotransplant mouse model of human glioma-initiating cells (hGICs) that generates a disseminated brain-tumour phenotype featuring an intact BBB. We demonstrate that all types of EVs of tumour cells — apoptotic bodies, shedding microvesicles, and exosomes — carry human genomic-DNA oncogene sequences and can cross the intact BBB and reach the bloodstream. These EVs can be isolated from peripheral blood and readily distinguished from total EVs based on their human-specific DNA sequences. Notably, these EVs carrying human oncogenic genomic-DNA (gDNA) sequences corresponding to those of the cells of origin can be isolated and enriched from peripheral blood (Figure 3).

### The BBB is intact in GBM1 xenograft model

Our aim was to examine the entry of EVs of glioma cells into the bloodstream through the normal BBB; thus, we used an orthotopic xenotransplant model of hGICs culture (GBM1) that generates a disseminated brain-tumour phenotype featuring an intact BBB in nude mice. First, we demonstrated the BBB integrity of this model by comparing it with a second orthotopic xenotransplant model of hGICs culture (GBM2) that generated a nodular brain tumour featuring a disrupted BBB. We performed stereotactic transplantations of 1 × 10⁵ cells from GBM1 and GBM2 into the striatum of mice, and 12 weeks later, validated the functional competence of the BBB by using three distinct procedures. For negative controls, we used the same method and injected 2 mice with PBS (Figure 4).

We first analysed the BBB of mice xenotransplanted with either GBM1 or GBM2 cells by using MRI (Figure 5a and Figure 4a). In the case of tumours generated by GBM2 cells, the T2-weighted sequence showed a cortical tumour exhibiting a mass effect on the left lateral ventricle. A well-defined mass displaying homogenous hyperintensity without significant surrounding vasogenic oedema was observed. Furthermore, T1 MRI post-contrast weighted images demonstrated a homogeneous enhancement, presumably due to contrast extravasation, a marker of BBB disruption. In contrast to the GBM2 tumour images, the T1 post-contrast weighted images obtained for GBM1 tumours showed no enhancement, which suggested that the BBB was not leaky, and the T2-weighted images showed diffuse hemispheric involvement, which demonstrated tumour projection into the contralateral hemisphere through the corpus callosum. To further test for a potential BBB damage in these two brain-tumour models, we visualised and quantified the amount of Evans Blue present within the parenchymal brain of mice after cells were injected through the tail vein. The results showed that Evans Blue extravasation was significantly increased in the brains of mice xenotransplanted with GBM2 cells as compared with that in the GBM1 case (p < 0.05) (Figure 5b,c), and that no Evans Blue dye was detected in the control brains (Figure 4b). Lastly, to demonstrate the functional competence of the BBB, albumin extravasation was analysed using immunofluorescence (Figure 5d): we observed considerable albumin diffusion from blood vessels into the parenchymal brain of mice xenotransplanted with GBM2 cells, but detected no albumin extravasation in the brains of either the mice xenotransplanted with GBM1 cells or the control mice (Figure 5e and Figure 4d).

Collectively, our results confirmed that the GBM1 orthotopic xenotransplant model of hGIC culture displays an intact BBB, which is appropriate for evaluating the possibility that brain-tumour EVs can cross an intact BBB to enter the bloodstream.

### Morphological characterisation and size distribution of EVs

After obtaining a suitable model, we tested whether all three types of EVs could be isolated and enriched from both the hGICs (GBM1) supernatant and mouse plasma. EVs were isolated and enriched using regular ultracentrifugation and then visualized using transmission electron microscopy (TEM) (Figure 6a and Figure 7a).

TEM images revealed the typical morphology and expected diameter ranges of the ABs (>1 µm), SMVs (~200 nm), and EXOs (~100 nm). To confirm the enrichment of the different fractions of EVs from GBM1 cells, we analysed them using a Zetasizer; the AB fraction ranged from 2000 to 500 nm in size, the SMV fraction showed an average size of 600 nm, and the EXO fraction displayed a main peak at 180 nm (Figure 6b). The supernatant also showed a small 10-nm peak compatible with the presence of proteins.

These results confirmed the utility of the protocol used for isolating EVs from the supernatant. Although these images and sizes were consistent with the presence of all three types of EVs, we also quantified the relative expression of tetraspanin CD63, a commonly used marker for EXOs. The results indicated similar CD63 expression in SMVs and EXOs and a considerably lower expression in ABs (Figure 6c). Lastly, the EV distribution was quantified using TEM: the EXO fraction was almost 4 times larger than the AB and SMV fractions in the hGICs supernatant (Figure 6d), and a similar trend was observed in the case of EVs isolated from mouse plasma (Figure 7b).

### EVs from hGICs contain gDNA

Next, to confirm the presence of gDNA within the EVs isolated from the hGICs supernatants, we first treated the EVs with DNase I in order to eliminate free DNA sequences and then extracted the gDNA from each type of EV. The presence of human gDNA was verified by using a collection of primers (see Table 3) that were specifically designed and validated to amplify human gDNA sequences (Figure 8).

| **GENE** | **PRIMER NAME** | **SEQUENCE** | **AMPLICON SIZE** |
|---|---|---|---|
| **TP53 NM_000546** | TP53e7_F | CTTTGAGGTGCGTGTTTGTG (SEQ ID NO: 10) | 423 |
| | TP53i8_R | Caaccaggagccattgtctt (SEQ ID NO: 11) | |
| **PTEN NM_000314** | PTENe2_F | GCAGAAAGACTTGAAGGCGT A (SEQ ID NO: 12) | 232 |
| | PTENi2_R | Aaatgaactgtaccccctgaa (SEQ ID NO: 13) | |
| **MDM2 NM_002392** | MDM2i1_F | ctcagAATCATCGGACTCAGG (SEQ ID NO: 14) | 330 |
| | MDM2i3_R | Gctgtgtgaatgcgtcaaat (SEQ ID NO: 15) | |
| **MDM4** | MDM4i2_F | atcagGTACGACCAAAACTGC | 154 |
| **NM_002393** | | (SEQ ID NO: 16) | |
| | MDM4i3_R | Ctgggcaaaagagggagact (SEQ ID NO: 17) | |
| **AKT1 NM_00101443 1** | AKT1i12_F | Gagggtgtgctgggagtg (SEQ ID NO: 18) | 163 |
| | AKT1e13_R | (SEQ ID NO: 19) | |
| **AKT3 NM_005465** | AKT3i13_F | Gccgtggaaacactgaagtaa (SEQ ID NO: 20) | 183 |
| | AKT3e14_R | GTAGGAAGCCGActgtggac (SEQ ID NO: 21) | |
| **CDK4 NM_000075** | CDK4i2_F | Gggttggtaggcattgagag (SEQ ID NO: 22) | 211 |
| | CDK4e3_R | TCCACCACTTGTCACCAGAA (SEQ ID NO: 23) | |
| **CDKN2A NM_000077** | CDKN2Ae2_ F | GCACCAGAGGCAGTAACCAT (SEQ ID NO: 24) | 208 |
| | CDKN2Ai2_ R | Agggcgatagggagactcag (SEQ ID NO: 25) | |
| **ERBB2 NM_004448** | erbb2e25_F | CCCTTGGACAGCACCTTCTA (SEQ ID NO: 26) | 205 |
| | erbb2i25_R | Ggtaagcagacagccacaca (SEQ ID NO: 27) | |
| **EGFR NM_005228** | EGFRi12_F | tgctgtgacccactctgtct (SEQ ID NO: 28) | 192 |
| | EGFRi13_R | Caacgcaaggggattaaaga (SEQ ID NO: 29) | |
| **ASCL1 NM_004316** | ASCL1i1_F | Cctccatctccctctaccac (SEQ ID NO: 30) | 320 |
| | ASCL1e2_R | GACTCAGGTCCCAGTTGCTC (SEQ ID NO: 31) | |
| **MGMT NM_002412** | MGMTi3_F | Tgtgtagatgcgtttcctgttt (SEQ ID NO: 32) | 226 |
| | MGMTe4_R | ATTGCTCCTCCCACTGCTC (SEQ ID NO: 33) | |
| **RB1 NM_000321** | RB1e25_F | GGAAGCAACCCTCCTAAACC (SEQ ID NO: 34) | 111 |
| | RB1i25_R | Ccatctcggctactggaaaa (SEQ ID NO: 35) | |
| **PIK3CA NM_006218** | PIK3CAe19_ F | GGCTCAAAGACAAGAACAAA GG (SEQ ID NO: 36) | 103 |
| | PIK3CAi19_ R | Ggaatacacaaacaccgacaga (SEQ ID NO: 37) | |
| **IDH1 NM_005896** | Idh1i3_F | Aggggaatgtctggacctct (SEQ ID NO: 38) | 245 |
| | Idh1e4_R | TCCGTCACTTGGTGTGTAGG (SEQ ID NO: 39) | |
| **IDH2** | IDH2e10_F | GAGAAGGTGTGCGTGGAGA | 266 |
| **NM_002168** | | (SEQ ID NO: 40) | |
| | IDH2i10_R | Tcagggatggggaggaac (SEQ ID NO: 41) | |
| | | | |
| **G3PDH** | hmG3PDH_ F | ACACACAGTCCATGCCATCA C (SEQ ID NO: 42) | 450 |
| | hmG3PDH_ R | TCCACCACCCTGTTGCTGTA (SEQ ID NO: 43) | |

Table 3: Primers used for confirming the presence of gDNA within the EVs isolated from the hGICs supernatants. The name of the primers relates to the target gene (F, Forward primer; R, Reverse primer).

We detected all the gDNA oncogene sequences assayed, except for CDKN2a, PTEN, TP53, and NF1 sequences (Figure 9a). We speculated that the lack of PCR-amplification of these gene sequences might be related to the existence of chromosomal aberrations. To test this possibility, we examined the chromosomal status of GBM1 cells by using comparative genomic hybridisation (CGH). The CGH analysis revealed loss of heterogeneity of chromosome 9 at p21.3, chromosome 10 at q23.1, and chromosome 17 at positions p13.1 and q11.2, which respectively affect the CDKN2a, PTEN, and TP53 and NF1 locations).

Intriguingly, gDNA oncogenic sequences were detected in all three types of EVs. Certain oncogenes appeared in all EVs, such as ERBB2, EGFR, CDK4, AKT3, and MDM2, whereas IDH1 was detected only in ABs and EXOs, and a few oncogenes appeared exclusively in one type of EV: PIK3CA, MDM4, IDH2, and ASCL1 in ABs, AKT1 and RB1 in SMVs, and MGMT in EXOs (Figure 9a-c). Nonetheless, we observed an 86% frequency of bias in this assay that was independent of our experimental procedure (Figure 10).

### hGIC-derived EVs cross the intact BBB into the bloodstream

Lastly, we used the GBM1 orthotopic xenotransplant model of hGICs culture to analyse whether all three types of EVs that are produced by GBM1 cells and carry human gDNA oncogenic sequences can cross the undisrupted BBB and reach the bloodstream. We hypothesised that if this was the case, we should be able to detect human gDNA oncogenic sequences within the pool of EVs (of both mouse and human origin) isolated from the peripheral blood. Thus, we isolated and separated all three types of EVs from the inferior vena cava, and then isolated total DNA from each type of EV and performed PCR assays to specifically amplify human gDNA oncogenic sequences. PCR-amplification products of the expected sizes were obtained for AKT3 from ABs, PIK3CA from ABs and SMVs, and MDM4 and EGFR from EXOs, and multiple sequence alignment showed a complete homology among these sequences and their counterparts from the supernatants of hGICs-enriched cultures (Figure 11).

Our results indicate that EV-based biomarkers can be used in the management of human gliomas. Lastly, the ability to separate EVs secreted by tumour cells from the total EVs pool is a critical factor that must be addressed in order to facilitate the use of EVs as biomarkers. Here, we have shown that orthotopic xenotransplant animal models of human tumour cells are suitable models for providing 'proof of concepts' related to the utility of specific molecular biomarkers present within EVs: our results revealed that both the surface molecules and the cargo present in EVs derived from human tumour cells could be readily distinguished from those of the host.

In conclusion, we have demonstrated that all three types of EVs secreted by human glioma cells can cross the BBB whether it is intact or disrupted. This finding highlights the potential for the use of circulating-EVs cargo as diagnostic, prognostic, and followup biomarkers for both low- and high-grade gliomas.

Therefore the present invention demonstrates that:
- Cold-PCR allows for the detection of IDH1R132H from total DNA when the concentration of IDH R132H DNA sequences is as low as 10% of total IDH1 sequences.
- IDH1R132H DNA sequences can be detected within EVs isolated from peripheral blood extracted from glioma patients.
- The sensitivity of Cold-PCR is higher than regular PCR to detect IDH1R132H DNA sequences within EVs isolated from peripheral blood extracted from glioma patients.
- The sensitivity and reproducibility of this technique is as good as regular PCR and immunohistochemistry applied on solid samples.

The results obtained here with IDH in glioma patients, can be extrapolated to other non-invasive samples and type of cancers. Moreover, the technique used here is an example of the allele-enriching techniques. The utilization of Cold-PCR (or allele-enriching techniques) on DNA sequences within EVs isolated from peripheral blood, might be also applied for the detection of any other gene sequence carrying point mutations, with clinical or biological value, including, but not limited to EGFR, BRAFT, KRAS, NRAS, MET, ALK, C-KIT, DDR2, ERBB2, ERBB3, ERBB4, FGFR2, IDH2, PALB2 PDGFR, PIK3CA, PTCH1, SMO, and Histone H3. This technology is especially valuable for the evaluation of biomarkers in pathologies where solid pathological sample is not available or minimally invasive approaches are necessary. These pathologies include but are not limited to inoperable tumours, inaccesible tumours or those for which biopsies or surgical removal of cancerous tissue is too risky.

## Claims

1. A method for detecting a gene mutation and/or a minority allele with a ratio below at least 1%, preferably below at least 0.1%, compared to the wild type gene or allele comprising:
a) extracting a nucleic acid and/or a protein from the extracellular vesicles isolated from a liquid sample of a subject, with the proviso that the liquid sample is not cerebrospinal fluid,
b) enriching of the mutation and/or the minority allele, and
c) identifying the gene mutation and/or the minority allele.

2. Method according to claim 1, wherein the liquid sample is selected from the group consisting of serum, plasma, blood, saliva, bronchoalveolar lavage fluid, milk, synovial fluid, amniotic fluid and urine.

3. Method according to any one of claims 1 or 2, wherein the subject suffers from a disease selected from the group consisting of glioma, lung, breast, prostate, melanoma, kidney, hepatic, pancreatic, colon, cervical, esophageal, bladder, head and neck cancer, sarcoma, lymphoma, leukemia, myeloma, malignant plasma cell disorder, meningioma, difuse intrinsic pontine gliomas (DIPG), neuroblastoma or medulloblastoma.

4. Method according to any one of claims 1 to 3, wherein the method for the enrichment of the minority allele and/or the mutation is selected from the group consisting of COLD-PCR, coamplification at lower denaturation temperature Polimerase Chain Reaction, ARMS, amplification refractory mutation system; ASPCR, allele-specific enzymatic amplification; ASA, allele-specific amplification; PASA, PCR amplification of specific alleles; PAMSA, PCR amplification of multiple specific alleles; COP, competitive oligonucleotide priming; E-PCR, enriched PCR; ME-PCR, mutant-enriched PCR; MAMA, mismatch amplification mutation assay; MASA, mutant allele-specific amplification; aQRT-PCR, antiprimer quenching-based real-time PCR; REMS-PCR, restriction endonuclease-mediated selective PCR; AIRS, artificial introduction of a restriction site; PNA, peptide nucleic acid; LNA, locked nucleic acid; WTB-PCR, wild-type blocking PCR; FLAG, fluorescent amplicon generation; RSM-PCR, restriction site mutation PCR; APRIL-ATM, amplification via primer ligation, at the mutation; PAP, pyrophosphate-activated polymerization; RMC, random mutation capture; CCM, chemical cleavage of mismatches; endo, endonuclease; TDG, thymine DNA glycosylase; CEL I, celery extract I; HRM, high-resolution melting; SSCP, single-strand conformation polymorphism; DGGE, denaturing gradient gel electrophoresis; CDCE, constant denaturing capillary electrophoresis; dHPLC, denaturing HPLC; and iFLP, inverse PCR-based amplified RFLP.

5. Method according to any one of claims 1 to 4, wherein the gene mutation is detected in the gene selected from the group consisting of IDH1, EGFR, BRAFT, KRAS, NRAS, MET, ALK, C-KIT, DDR2, ERBB2, ERBB3, ERBB4, FGFR2, IDH2, PALB2 PDGFR, PIK3CA, PTCH1, SMO and Histone H3 gene.

6. Method according to claim 5 wherein the gene is the IDH1 gene.

7. Method according to claim 6 wherein the mutation in the IDH1 gene is selected at least in the position 6745, 6746 or 6747, which correspond in the messenger RNA to positions 394, 395 and 396 respectively, and combinations thereof.

8. Method according to claim 7 wherein the mutation in the IDH1 gene results in a mutation in the IDH1 protein in position 132.

9. Method according to claim 8 wherein the mutation in the IDH1 gene results in a mutation in the IDH1 protein selected from R132H, R132C, R132S, R132G or R132L.

10. Method according to any one of claims 5 to 9, wherein in the step (b) the enrichment is performed using the pair of primers SEQ ID NO: 4 (5'-CGGTCTTCAGAGAAGCCATT-3') and SEQ ID NO: 5 (5'-GCAAAATCACATTATTGCCAAC-3'), or the pair of primers SEQ ID NO: 6 (5'- GTTGAAACAAATGTGGAAATC - 3') and SEQ ID NO: 7 (5'-TAATGGGTGTAGATACCAAAAG- 3').

11. Method according to any one of claims 6 to 10 wherein the gene mutation is detected in IDH1 gene, it is detected by Cold-PCR comprising the following steps:
a. a denaturation step of 90-99°C, preferably 96°C, during 5 to 15 minutes, preferably 10 minutes;
b. a following step of 15 to 35 cycles, preferably 20 cycles, comprising a denaturation step of 90-98°C, preferably 96°C, during 10-20 seconds, preferably 15 seconds; and an annealing and primer extension step of 55-65°C, preferably 60°C, during 10-20 seconds, preferably 15 seconds;
c. a next step of 15 to 35 cycles, preferably 30 cycles, comprising a denaturation step (critical temperature) of 70-85°C, preferably79°C, during 10-20 seconds, preferably 15 seconds; and an annealing and primer extension step of 55-65°C, preferably 60°C, during 10-20 seconds, preferably 15 seconds; and
d. a final step wherein the amplified DNA is maintained at a temperature below 20°C.

12. An *in vitro* method for the prognosis and/or the diagnosis of a disease associated with a gene mutation and/or a minority allele with a ration below at least 1% in a subject comprising detecting the presence of the mutation and/or the minority allele associated with the disease by a method according to any one of claims 1 to 11, wherein the detection of said mutation and/or the minority allele is indicative that the subject suffers from, or is going to suffer from said disease, or the detection of said mutation is indicative of good or bad prognosis of said disease.

13. Method according to claim 12, wherein the disease associated with a gene mutation is selected from the group consisting of glioma, lung, breast, prostate, melanoma, kidney, hepatic, pancreatic, colon, cervical, esophageal, bladder, head and neck cancer, sarcoma, lymphoma, leukemia, myeloma, malignant plasma cell disorder, meningioma, difuse intrinsic pontine gliomas (DIPG), neuroblastoma or medulloblastoma.

14. Method according to claim 13, wherein the cancer is glioma, preferably the glioma is selected from the group consisting of astrocytoma, oligodendroglyoma and oligoastrocytoma.

15. Method according to claim 14, wherein if the mutation detected is in the gene IDH1, it encodes an IDH1 protein with a mutation in position 132, preferably R132H, R132C y/o R132G, then the subject has good prognosis.
